# EUROPEAN PATENT SPECIFICATION

(11) **EP 0 923 556 B1**
(45) Date of publication and mention of the grant of the patent: **08.10.2003**
(21) Application number: 97936396.7
(22) Date of filing: 05.08.1997
(51) Int. Cl.: C07D 213/02, C07D 401/04, C07D 401/06, A61K 31/335, A61K 31/415, A61K 31/42, A61K 31/44

(54) **AN ASYMMETRIC CONJUGATE ADDITION REACTION**
ADDITIONSREAKTION MIT ASYMMETRISCHEM KONJUGAT
REACTION D'ADDITION PAR CONJUGUE ASYMETRIQUE

(30) Priority: 09.08.1996 US 23619 P; 28.08.1996 GB 9617949; 10.10.1996 US 28233 P; 25.11.1996 GB 9624467
(43) Date of publication of application: 23.06.1999
(73) Proprietor: MERCK & CO., INC., Rahway, New Jersey 07065 (US)
(72) Inventor: DOLLING, Ulf, H., Rahway, NJ 07065 (US); FREY, Lisa, F., Rahway, NJ 07065 (US); TILLYER, Richard, D., Rahway, NJ 07065 (US); TSCHAEN, David, M., Rahway, NJ 07065 (US)
(74) Representative: Hiscock, Ian James
(86) International application number: US9713725
(87) International publication number: WO98006698

(56) References cited:
- WO-A-93/08799
- WO-A-98/06700
- US-A- 5 017 608
- ALEXAKIS A ET AL: "Stereochemical effects of trimethylsilyl chloride (TMSCl) on the conjugate addition of organocopper reagents" TETRAHEDRON LETT. (TELEAY,00404039);1990; VOL.31 (3); PP.345-8, XP002118988 Univ. Pierre et Marie Curie;Lab. Chim. Organoelem.; Paris; 75252; Fr. (FR)
- FREY L F ET AL: "Stereoselective conjugate addition reactions of.alpha.,.beta.-unsaturated tert-butyl esters with aryllithium reagents" J. ORG. CHEM. (JOCEAH,00223263);1998; VOL.63 (9); PP.3120-3124, XP002118989 Merck Co. Inc.;Department of Process Research Merck Research Laboratories; Rahway; 07065; NJ; USA (US)
- ALEXAKIS A ET AL: "Diastereoselective conjugate addition with acetals. Oxazolidines and imidazolidines as chiral auxiliaries" TETRAHEDRON LETT. (TELEAY,00404039);1988; VOL.29 (35); PP.4411-14, XP002118990 Univ. P. et M. Curie;Lab. Chim. Org. Elements; Paris; F-75252/05; Fr. (FR)
- TETRAHEDRON LETTERS, 1988, Vol. 29, No. 35, ALEXAKIS et al., "Diastereoselective Conjugate Addition with Acetals, Oxazolidines and Imidazolines as Chiral Auxiliaries", pages 4411-4414.

## Description

### BACKGROUND OF THE INVENTION

The present invention relates to novel key intermediates in the synthesis of an endothelin antagonist and the method for preparing these key intermediates of formula I.

Two endothelin receptor subtypes ET_{A} and ET_{B} are known so far. The compounds of the present invention possess high affinity to at least one of two receptor subtypes, responsible for the dilation of smooth muscle, such as blood vessels or in the trachea. The endothelin antagonist compounds of the present invention provide a new therapeutic potential, particularly for the treatment of hypertension, pulmonary hypertension, Raynaud's disease, acute renal failure, myocardial infarction, angina pectoris, cerebral infarction, cerebral vasospasm, arteriosclerosis, asthma, gastric ulcer, diabetes, restenosis, prostatauxe endotoxin shock, endotoxin-induced multiple organ failure or disseminated intravascular coagulation, and/or cyclosporin-induced renal failure or hypertension.

Endothelin is a polypeptide composed of amino acids, and it is produced by vascular endothelial cells of human or pig. Endothelin has a potent vasoconstrictor effect and a sustained and potent pressor action (Nature, 332, 411-415 (1988)).

Three endothelin isopeptides (endothelin-1, endothelin-2 and endothelin-3), which resemble one another in structure, exist in the bodies of animals including human, and these peptides have vasoconstriction and pressor effects (Proc. Natl. Acad, Sci, USA, 86, 2863-2867 (1989)).

As reported, the endothelin levels are clearly elevated in the blood of patients with essential hypertension, acute myocardial infarction, pulmonary hypertension, Raynaud's disease, diabetes or atherosclerosis, or in the washing fluids of the respiratory tract or the blood of patients with asthmaticus as compared with normal levels (Japan, J. Hypertension, 12, 79, (1989), J. Vascular medicine Biology, 2, 207 (1990), Diabetologia, 33, 306-310 (1990), J. Am. Med. Association, 264, 2868 (1990), and The Lancet, ii, 747-748 (1989) and ii, 1144-1147 (1990)).

Further, an increased sensitivity of the cerebral blood vessel to endothelin in an experimental model of cerebral vasospasm (Japan. Soc. Cereb. Blood Flow & Metabol., 1, 73 (1989)), an improved renal function by the endothelin antibody in an acute renal failure model (J. Clin, invest., 83, 1762-1767 (1989), and inhibition of gastric ulcer development with an endothelin antibody in a gastric ulcer model (Extract of Japanese Society of Experimental Gastric Ulcer, 50 (1991)) have been reported. Therefore, endothelin is assumed to be one of the mediators causing acute renal failure or cerebral vasospasm following subarachnoid hemorrhage.

Further, endothelin is secreted not only by endothelial cells but also by tracheal epithelial cells or by kidney cells (FEBS Letters, 255, 129-132 (1989), and FEBS Letters, 249, 42-46 (1989)).

Endothelin was also found to control the release of physiologically active endogenous substances such as renin, atrial natriuretic peptide, endothelium-derived relaxing factor (EDRF), thromboxane A₂, prostacyclin, noradrenaline, angiotensin II and substance P (Biochem. Biophys, Res. Commun., 157, 1164-1168 (1988); Biochem. Biophys, Res. Commun., 155, 20 167-172 (1989); Proc. Natl. Acad. Sci. USA, 85 1 9797-9800 (1989); J. Cardiovasc. Pharmacol., 13, S89-S92 (1989); Japan. J. Hypertension, 12, 76 (1989) and Neuroscience Letters, 102, 179-184 (1989)). Further, endothelin causes contraction of the smooth muscle of gastrointestinal tract and the uterine smooth muscle (FEBS Letters, 247, 337-340 (1989); Eur. J. Pharmacol., 154, 227-228 (1988); and Biochem. Biophys Res. Commun., 159, 317-323 (1989)). Further, endothelin was found to promote proliferation of rat vascular smooth muscle cells, suggesting a possible relevance to the arterial hypertrophy (Atherosclerosis, 78, 225-228 (1989)). Furthermore, since the endothelin receptors are present in a high density not only in the peripheral tissues but also in the central nervous system, and the cerebral administration of endothelin induces a behavioral change in animals, endothelin is likely to play an important role for controlling nervous functions (Neuroscience Letters, 97, 276-279 (1989)). Particularly, endothelin is suggested to be one of mediators for pain (Life Sciences, 49, PL61-PL65 (1991)).

Internal hyperplastic response was induced by rat carotid artery balloon endothelial denudation. Endothelin causes a significant worsening of the internal hyperplasia (J. Cardiovasc. Pharmacol., 22, 355 - 359 & 371 - 373(1993)). These data support a role of endothelin in the phathogenesis of vascular restenosis. Recently, it has been reported that both ET_{A} and ET_{B} receptors exist in the human prostate and endothelin produces a potent contraction of it. These results suggest the possibility that endothelin is involved in the pathophysiology of benign prostatic hyperplasia (J. Urology, 151, 763 - 766(1994), Molecular Pharmocol., 45, 306 - 311(1994)).

On the other hand, endotoxin is one of potential candidates to promote the release of endothelin. Remarkable elevation of the endothelin levels in the blood or in the culture supernatant of endothelial cells was observed when endotoxin was exogenously administered to animals or added to the culture endothelial cells, respectively. These findings suggest that endothelin is an important mediator for endotoxin-induced diseases (Biochem. Biophys. Commun., 161, 1220-1227 (1989); and Acta Physiol. Scand., 137, 317-318 (1989)).

Further, it was reported that cyclosporin remarkably increased endothelin secretion in the renal cell culture (LLC-PKL cells) (Eur. J. Pharmacol., 180, 191-192 (1990)). Further, dosing of cyclosporin to rats reduced the glomerular filtration rate and increased the blood pressure in association with a remarkable increase in the circulating endothelin level. This cyclosporin-inducea renal failure can be suppressed by the administration of endothelin antibody (Kidney Int., 37, 1487-1491 (1990)). Thus, it is assumed that endothelin is significantly involved in the pathogenesis of the cyclosporin-induced diseases.

Such various effects of endothelin are caused by the binding of endothelin to endothelin receptors widely distributed in many tissues (Am. J. Physiol., 256, R856-R866 (1989)).

It is known that vasoconstriction by the endothelins is caused via at least two subtypes of endothelin receptors (J. Cardiovasc. Pharmacol., 17(Suppl.7), S119-S121 (1991)). One of the endothelin receptors is ETA receptor Selective to ET-1 rather than ET-3, and the other is ETB receptor equally active to ET-1 and ET-3. These receptor proteins are reported to be different from each other (Nature, 348, 730-735 (1990)).

These two subtypes of endothelin receptors are differently distributed in tissues. It is known that the ET_{A} receptor is present mainly in cardiovascular tissues, whereas the ET_{B} receptor is widely distributed in various tissues such as brain, kidney, lung, heart and vascular tissues.

Substances which specifically inhibit the binding of endothelin to the endothelin receptors are believed to antagonize various pharmacological activities of endothelin and to be useful as a drug in a wide field. Since the action of the endothelins is caused via not only the ET_{A} receptor but also the ET_{B} receptor, novel non-peptidic substances with ET receptor antagonistic activity to either receptor subtype are desired to block activities of the endothelins effectively in various diseases.

Endothelin is an endogenous substance which directly or indirectly (by controlling liberation of various endogenous substances) induces sustained contraction or relaxation of vascular or non-vascular smooth muscles, and its excess production or excess secretion is believed to be one of pathogeneses for hypertension, pulmonary hypertension, Raynaud's disease, bronchial asthma, gastric ulcer, diabetes, arteriosclerosis, restenosis, acute renal failure, myocardial infarction, angina pectoris, cerebral vasospasm and cerebral infarction. Further, it is suggested that endothelin serves as an important mediator involved in diseases such as restenosis, prostatauxe, endotoxin shock, endotoxin-induced multiple organ failure or disseminated intravascular coagulation, and cyclosporin-induced renal failure or hypertension. Two endothelin receptors ET_{A} and ET_{B} are known so far. An antagonistic agent against the ET_{B} receptor as well as the ET_{A} receptor is useful as a drug. In the field of anti-endothelin agents, some non-peptidic compounds possessing antagonistic activity against endothelin receptors were already disclosed in patents (for example, EP 0526708 A1, WO 93/08799 A1).

Alexakis et al. (*Tetrahedron Lett.,* 1990, **31**(3), 345-348) is directed to the diastereoselective conjugate addition of organocopper reagents to cinnamates bearing a chiral imidazolidine or oxazolidine ring.

Devine et al. (WO 98/06700) discloses a key intermediate in the synthesis of an endothelin antagonist, the synthesis of the key intermediate and the synthesis of an endothelin antagonist using this intermediate in a stereoselective deoxygenation reaction.

Cousins et al. (WO 93/08799) discloses indane and indene derivatives which are endothelin receptor antagonists.

Frey et al. (*J. Org. Chem.,* **63**(9), 3120-3124) is related to the reaction of aryllithium reagents with α,β-unsaturated *tert*-butyl esters possessing chiral imidaxolidine or oxazolidine auxiliaries to develop a general method for enantioselective synthesis of chiral β,β-diarylpropanonates.

Alexakis et al. (*Tetrahedron Lett.,* 1988, **29**(35), 4411-4414) involves a stereo selective 1,4-addition of lithium diphenyl cuprate to α,β-unsaturated ethyl esters bearing a chiral oxazolidine or imidazolidine ring.

Accordingly, it is an object of the present invention to provide novel key intermediates in the synthesis of an endothelin antagonist and a process for preparing the novel intermediates.

In order to accomplish the above object, the present inventors have developed an asymmetric conjugate addition which enables them to prepare the compound of Formula I, 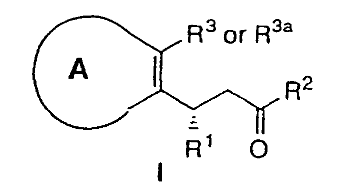 a key intermediate in the synthesis of endothelin antagonists of the following structure: 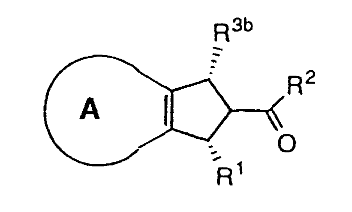 wherein 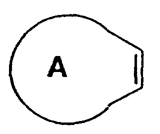 represents: 5- or 6-membered heterocyclyl, 5- or 6-membered carbocyclyl, and aryl;
- R^{3b}: is aryl , or heteroaryl;
- R¹: is: C₁-C₈ alkyl, C₂-C₈ alkenyl, C₂-C₈ alkynyl, C₃-C₈ cycloalkyl, aryl, or heteroaryl;
- R²: is OR⁴ and N(R⁵)₂;
- R⁴: is C₁-C₈ alkyl; and
- R⁵: is: C₁-C₈ alkyl, or aryl.

### SUMMARY OF THE INVENTION

This invention relates to a key intermediate in the synthesis of an endothelin antagonist and the synthesis of this key intermediate using an asymmetric conjugate addition reaction.

The instant invention relates to a compound of formula I: 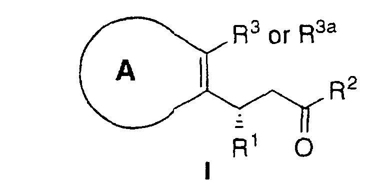 wherein 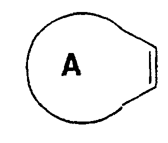 represents:
a) 5- or 6-membered heterocyclyl containing one, two or three double bonds, but at least one double bond and 1, 2 or 3 heteroatoms selected from O, N and S, the heterocyclyl is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
b) 5- or 6-membered carbocyclyl containing one or two double bonds, but at least one double bond, the carbocyclyl is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
c) aryl, wherein aryl is as defined below,
C₁-C₈ alkoxy, C₁-C₈ alkyl, C₂-C₈ alkenyl, C₂-C₈ alkynyl, or C₃-C₈ cycloalkyl, are unsubstituted or substituted with one, two or three substituents selected from the group consisting of: OH, CO₂R⁴, Br, Cl, F, I, CF₃, N(R⁵)₂, C₁-C₈ alkoxy, C₃-C₈ cycloalkyl, CO(CH₂)ₙCH₃, and CO(CH₂)ₙCH₂N(R⁵)₂,
aryl is defined as phenyl or naphthyl , which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, CO(CH₂)ₙCH₂N(R⁵)₂, and when two substituents are located on adjacent carbons they can join to form a 5- or 6-membered ring with one, two or three heteroatoms selected from O, N, and S, which is unsubstituted or substituted with with one, two or three substituents selected from the group consisting of: H, OH, CO₂R⁶, Br, Cl, F, I, CF₃, N(R⁷)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
n is 0 to 5;
- R¹: is:
a) C₁-C₈ alkyl, C₂-C₈ alkenyl, C₂-C₈ alkynyl, C₃-C₈ cycloalkyl,
b) aryl, or
c) heteroaryl;
heteroaryl is defined as a 5- or 6-membered aromatic ring containing 1, 2 or 3 heteroatoms selected from O, N and S , which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
- R²: is OR⁴ or N(R⁵)₂;
- R³: is 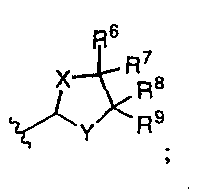
- R^{3a}: is:
a) CHO,
b) -CO-C₁-C₈ alkyl,
c) -CO-aryl, or
d) -CO-heteroaryl;
- X and Y: are independently: O, S, or NR⁵;
- R⁴: is branched C₃-C₈ alkyl;
- R⁵: is: C₁-C₈ alkyl, or aryl; and
- R⁶, R⁷, R⁸ and R⁹: are independently: H, C₁-C₈ alkyl, and aryl, such that either R⁶ and R⁷ are not the same and/or R⁸ and R⁹ are not the same, or R⁶ and R⁸ or R⁷ and R⁹ can join to form a 5- or 6-membered ring, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, CO(CH₂)ₙCH₂N(R⁵)₂.

Also within the scope of the instant invention is a process for the preparation of a compound of formula I: 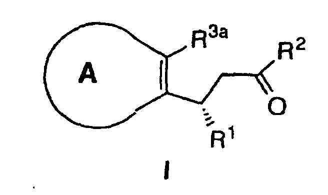 wherein the substituents are as defined above, comprising reacting a α,β-unsaturated ester or amide 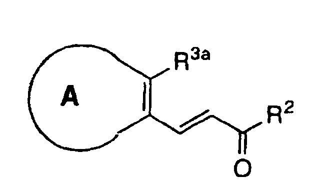 with an organolithium compound, R¹Li, in the presence of an aprotic solvent at a temperature range of about -78°C to about 0°C.

Also within the scope of the instant invention is a process for the preparation of the compound of formula I: 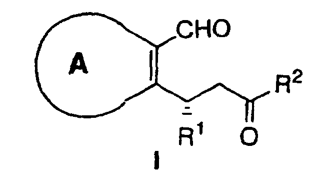 wherein the substituents are as defined above, comprising the steps of:
1) reacting an α,β-unsaturated ester or amide 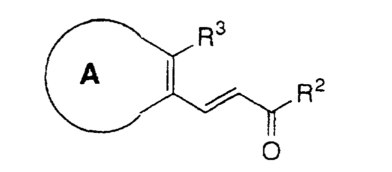 with an organolithium compound, R¹Li, in the presence of an aprotic solvent at a temperature range of about -78°C to about 0°C to give the conjugate adduct; and
2) removing the chiral auxiliary, R³, with aqueous acid and tetrahydrofuran to give the compound of Formula I.

### DETAILED DESCRIPTION OF THE INVENTION

The instant invention relates to a compound of formula I: 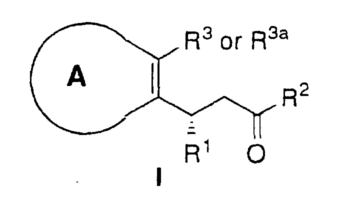 wherein 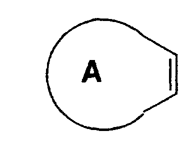 represents:
a) 5- or 6-membered heterocyclyl containing one, two or three double bonds, but at least one double bond and 1, 2 or 3 heteroatoms selected from O, N and S, the heterocyclyl is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
b) 5- or 6-membered carbocyclyl containing one or two double bonds, but at least one double bond, the carbocyclyl is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
c) aryl, wherein aryl is as defined below,
C₁-C₈ alkoxy, C₁-C₈ alkyl, C₂-C₈ alkenyl, C₂-C₈ alkynyl, or C₃-C₈ cycloalkyl, are unsubstituted or substituted with one, two or three substituents selected from the group consisting of: OH, CO₂R⁴, Br, Cl, F, I, CF₃, N(R⁵)₂, C₁-C₈ alkoxy, C₃-C₈ cycloalkyl, CO(CH₂)ₙCH₃, and CO(CH₂)ₙCH₂N(R⁵)₂,
aryl is defined as phenyl or naphthyl, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, CO(CH₂)ₙCH₂N(R⁵)₂, and when two substituents are located on adjacent carbons they can join to form a 5- or 6-membered ring with one, two or three heteroatoms selected from O, N, and S, which is unsubstituted or substituted with one, two or three substituents selected from the group consisting of: H, OH, CO₂R⁶, Br, Cl, F, I, CF₃, N(R⁷)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
n is 0 to 5;
- R¹: is:
a) C₁-C₈ alkyl, C₂-C₈ alkenyl, C₂-C₈ alkynyl, C₃-C₈ cycloalkyl,
b) aryl, or
c) heteroaryl;
heteroaryl is defined as a 5- or 6-membered aromatic ring containing 1, 2 or 3 heteroatoms selected from O, N and S, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
- R²: is OR⁴ or N(R⁵)₂;
- R³: is 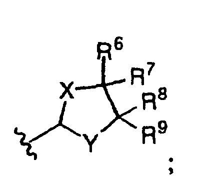
- R^{3a}: is:
a) CHO,
b) -CO-C₁-C₈ alkyl,
c) -CO-aryl, or
d) -CO-heteroaryl;
- X and Y: are independently: O, S, or NR⁵;
- R⁴: is branched C₃-C₈ alkyl;
- R⁵: is: C₁-C₈ alkyl, or aryl; and
- R⁶, R⁷, R⁸ and R⁹: are independently: H, C₁-C₈ alkyl, and aryl, such that either R⁶ and R⁷ are not the same and/or R⁸ and R⁹ are not the same, or R⁶ and R⁸ or R⁷ and R⁹ can join to form a 5- or 6-membered ring, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, CO(CH₂)ₙCH₂N(R⁵)₂.

The instant invention also relates to a process for the preparation of a compound of formula I: 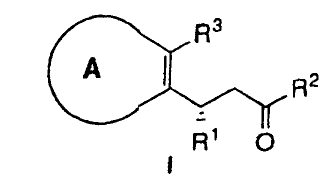 wherein 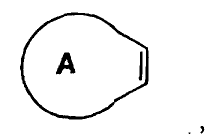 R¹, R² and R³ are as previously defined in connection with formula I;
comprising reacting a α,β-unsaturated ester or amide 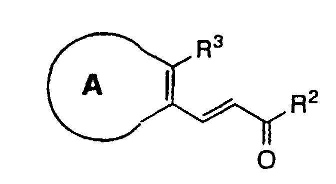 with an organolithium compound, R¹Li, in the presence of an aprotic solvent at a temperature range of about -78°C to about 0°C.

The process as recited above, wherein the number of equivalents of the organolithium compound, R¹Li, is 1 to about 4. The process as recited above, wherein the aprotic solvent is selected from the group consisting of tetrahydrofuran, diethyl ether, MTBE (methyl t-butyl ether), toluene, benzene, pentane, hexane, dioxane or a mixture of said solvents. The process as recited above, wherein the temperature range is about -78°C to about -20°C, and preferably about -78°C to about -50°C.

An embodiment of this invention is the process for the preparation of a compound of formula I: 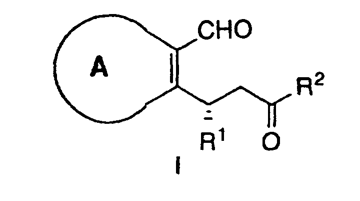 wherein 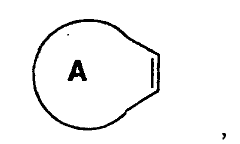 R¹ and R² are as previously defined in connection with formula I;
comprising the steps of:
1) reacting an α,β-unsaturated ester or amide 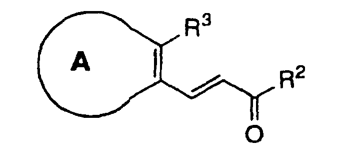 with an organolithium compound, R¹Li, in the presence of an aprotic solvent at a temperature range of about -78°C to about 0°C to give the conjugate adduct; and
2) removing the chiral auxiliary, R³, with aqueous acid and tetrahydrofuran to give the compound of Formula I.

The process as recited above, wherein the number of equivalents of the organolithium compound, R¹Li, is 1 to about 4. The process as recited above, wherein the aprotic solvent is selected from the group consisting of tetrahydrofuran, diethyl ether, MTBE (methyl t-butyl ether), toluene, benzene, hexane, pentane, dioxane or a mixture of said solvents. The process as recited above, wherein the temperature range is about -78°C to about -20°C, and preferably about -78°C to about -50°C. The process as recited above, wherein the aqueous acid is aqueous acetic acid.

An embodiment of this invention is the process for the preparation of an aldehyde 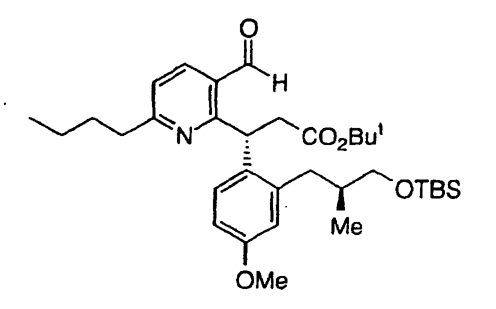 comprising reacting an α,β-unsaturated ester 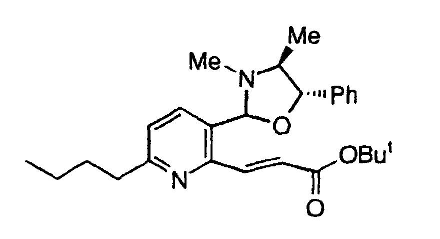 with an organolithium compound 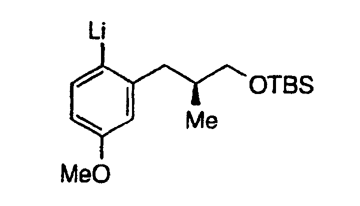 in the presence of an aprotic solvent at a temperature range of about -78°C to about -20°C.

The process as recited is above, wherein the number of equivalents of the organolithium compound, R¹Li, is 1 to about 4 and preferably is 1.5 to about 2.5 equivalents. The process as recited above, wherein the aprotic solvent is selected from the group consisting of tetrahydrofuran, diethyl ether, methyl t-butyl ether (MTBE), toluene, benzene, hexane, pentane, dioxane or a mixture of said solvents, and the preferable aprotic solvent is tetrahydrofuran. The process as recited above, wherein the preferred temperature range is about -78°C to about -50°C and a more preferred range is about -78°C to about -70°C.

A second embodiment of this invention is the process for the preparation of a ketone of formula: 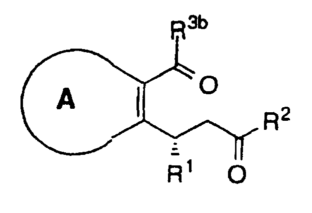 wherein 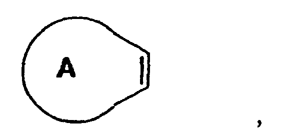 R¹ and R² are as previously defined in connection with formula I; and
R^{3b} is:
a) C₁-C₈ alkyl,
b) aryl, or
c) heteroaryl;
comprising the steps of:
1) reacting a α,β-unsaturated ester or amide 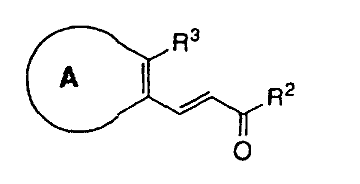 with an organolithium compound, R¹Li, in the presence of an aprotic solvent at a temperature range of about -78°C to about 0°C to give a conjugate adduct 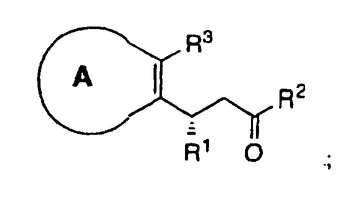
2) removing the chiral auxiliary with aqueous acid and tetrahydrofuran to give the aldehyde 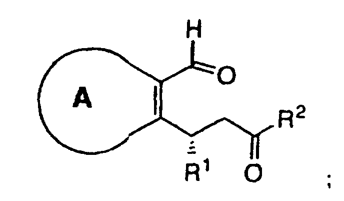
3) reacting the aldehyde with a Grignard reagent or organolithium reagent formed with R^{3b}Z, where Z is Br, Cl, or I to form an alcohol 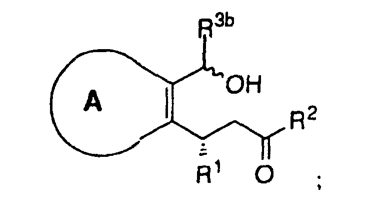
4) oxidizing the alcohol formed with an oxidizing agent to give the ketone

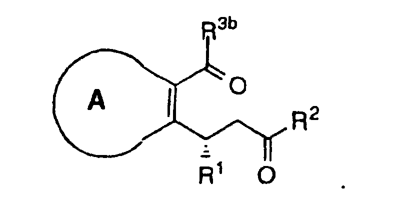

The process as recited above, wherein the number of equivalents of the organolithium compound, R¹Li, in the first step is 1 to about 4. The process as recited above, wherein the aprotic solvent in the first step is selected from the group consisting of tetrahydrofuran, diethyl ether, methyl t-butyl ether, toluene, benzene, pentane, hexane, dioxane or a mixture of said solvents. The process as recited above, wherein the temperature range in the first step is about -78°C to about -70°C.

The process as recited above, wherein the aqueous acid in the second step is aqueous acetic acid.

The process as recited above, wherein the oxidizing agent in the fourth step is 4-methylmorpholine-N-oxide (NMO) and tetrapropylammonium perruthenate(VII) (TPAP).

A second embodiment of this invention is the process for the preparation of a ketone of formula: 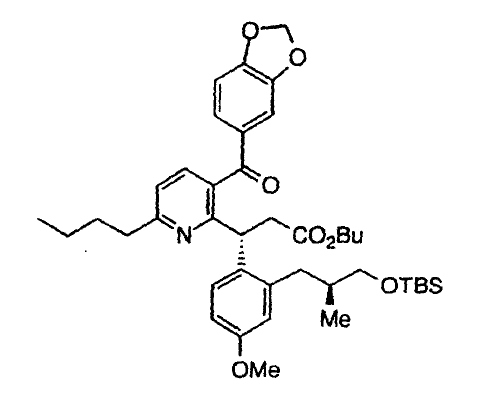 comprising the steps of:
1) reacting a α,β-unsaturated ester 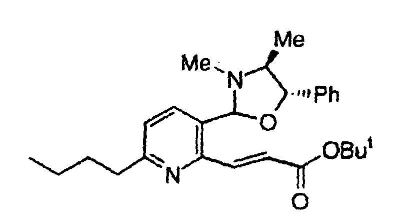 with an organolithium compound 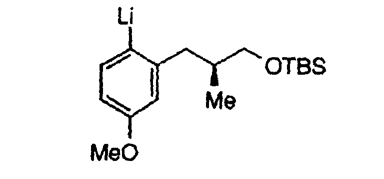 in the presence of an aprotic solvent at a temperature range of about -78°C to about 0°C to give a conjugate adduct 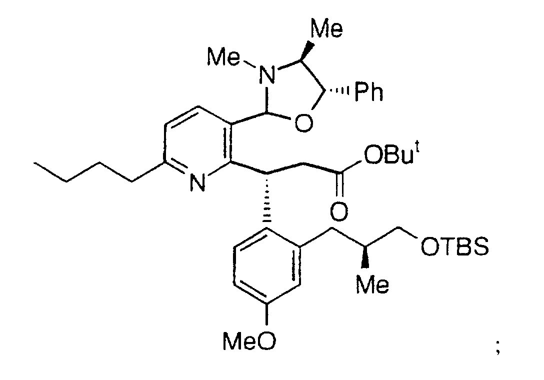
2) removing the chiral auxiliary with aqueous acid and tetrahydrofuran to give the aldehyde 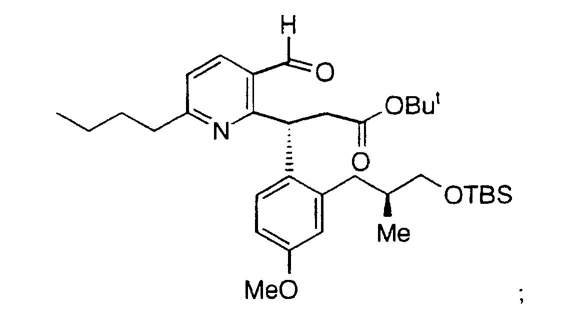
3) reacting the aldehyde with a Grignard reagent or organolithium reagent formed with 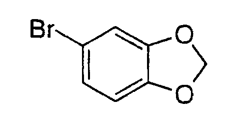 to form an alcohol 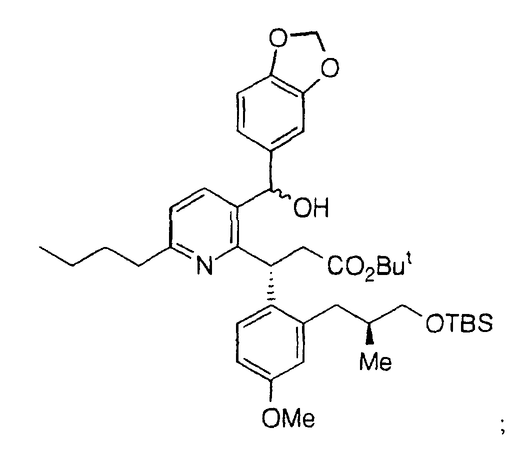
4) oxidizing the alcohol formed with an oxidizing agent to give a ketone 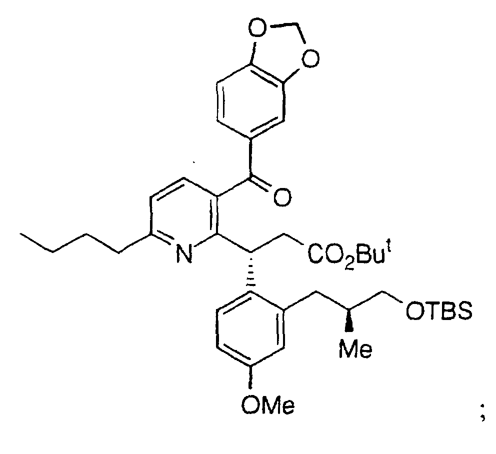 and
5) transesterifying the ester with n-butanol and a Lewis acid to give the desired n-butyl ester.

The process as recited above, wherein the number of equivalents of the organolithium compound, R¹Li, in the first step is 1 to about 4. The process as recited above, wherein the aprotic solvent in the first step is selected from the group consisting of tetrahydrofuran, diethyl ether, methyl t-butyl ether, toluene, benzene, pentane, hexane, dioxane or a mixture of said solvents. The process as recited above, wherein the temperature range in the first step is about -78°C to about -50°C.

The process as recited above, wherein the aqueous acid in the second step is aqueous acetic acid.

The process as recited above, wherein the Grignard reagent or organolithium reagent in the third step are 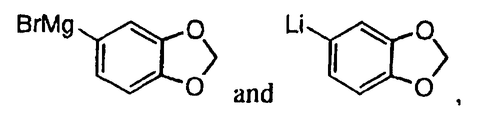 respectively.

The process as recited above, wherein the oxidizing agent in the fourth step is 4-methylmorpholine-N-oxide (NMO) and tetrapropylammonium perruthenate(VII) (TPAP).

The process as recited above, wherein the fifth step is conducted in the presence of a Lewis acid selected from Ti(OEt)₄, Ti(OiPr)₄, or Ti(OBu)₄.

It is further understood that the substituents recited above would include the definitions recited below.

The alkyl substituents recited above denote straight and branched chain hydrocarbons of the length specified such as methyl, ethyl, isopropyl, isobutyl, tert-butyl, neopentyl, isopentyl, etc.

The alkenyl-substituents denote alkyl groups as described above which are modified so that each contains a carbon to carbon double bond such as vinyl, allyl and 2-butenyl.

Cycloalkyl denotes rings composed of 3 to 8 methylene groups, each of which may be substituted or unsubstituted with other hydrocarbon substituents, and include for example cyclopropyl, cyclopentyl, cyclohexyl and 4-methylcyclohexyl.

The alkoxy substituent represents an alkyl group as described above attached through an oxygen bridge.

The heteroaryl substituent represents an carbazolyl, furanyl, thienyl, pyrrolyl, isothiazolyl, imidazolyl, isoxazolyl, thiazolyl, oxazolyl, pyrazolyl, pyrazinyl, pyridyl, pyrimidyl, purinyl.

The heterocyclyl substituent represents a pyridyl, pyrimidyl, thienyl, furanyl, oxazolidinyl, oxazolyl, thiazolyl, isothiazolyl, pyrazolyl, triazolyl, imidazolyl, imidazoldinyl, thiazolidilnyl, isoxazolyl, oxadiazolyl, thiadiazolyl, morpholinyl, piperidinyl, piperazinyl, pyrrolyl, or pyrrolidinyl.

The α,β-unsaturated ester or amide 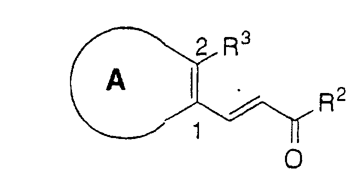 can generally be prepared in two steps:
1) a coupling reaction at the one position of Ring A 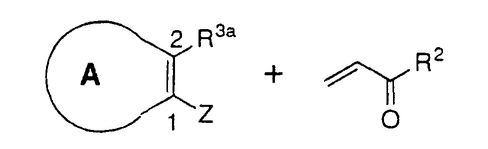 wherein Z is a leaving such as Br, Cl, I, OTriflyl, OTosyl or OMesyl and R² is OR⁴ or N(R⁵)₂; and
2) the conversion of the aldehyde (R^{3a}=CHO) to the desired chiral auxiliary (R³), wherein R³ represents 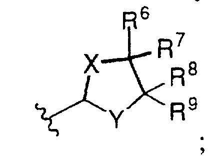 X and Y are independently: O, S, or NR⁵; R⁴ is C₁-C₈ alkyl; R⁵ is: C₁-C₈ alkyl, or aryl; and R⁶, R⁷, R⁸ and R⁹ are independently: H, C₁-C₈ alkyl, and aryl, such that either R⁶ and R⁷ are not the same and/or R⁸ and R⁹ are not the same, or R⁶ and R⁸ or R⁷ and R⁹ can join to form a 5- or 6-membered ring, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, CO(CH₂)ₙCH₂N(R⁵)₂.

Commercially available pyridone **1** is alkylated via its dianion with propyl bromide, and the product is then converted into the bromopyridine **3a** using a brominating agent such as PBr₃. The nitrile **3a** is then reduced to the aldehyde **3** using diisobutyl aluminum hydride (DIBAL). The aldehyde then undergoes a Heck reaction with t-butyl acrylate using NaOAc, (allyl)₂PdCl₂, tri-o-tolylphosphine, toluene, reflux to provide the unsaturated ester **4a** in high yield. The unsaturated ester **4a** is then reacted with a chiral auxiliary to give the acceptor **5a**. Examples of chiral auxiliaries useful in this method are the enantiomers of pseudoephedrine, ephedrine, 1N,2N-dimethyldiaminocyclohexane, diphenylprolinol, N-methylaminoindanol, and 1N,2N-diethyldiaminocyclohexane.

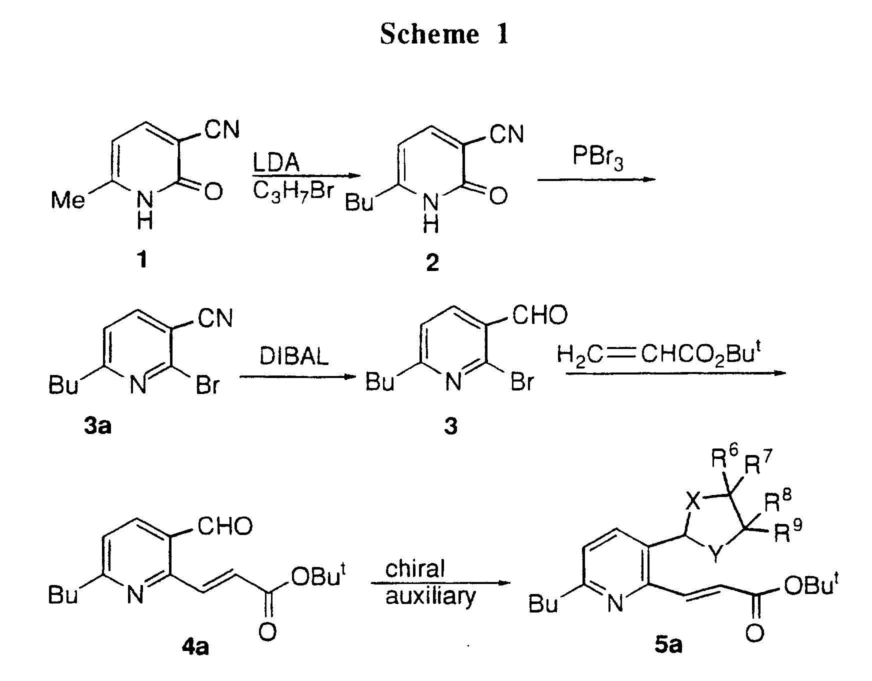

Commericially available acid **10** is reduced with BH₃•SMe₂, to the alcohol **11**, which is then converted into the bromide**13**, via the mesylate **12** using mesyl chloride, triethylamine followed by the addition of NaBr and dimethylacetamide (DMAC).

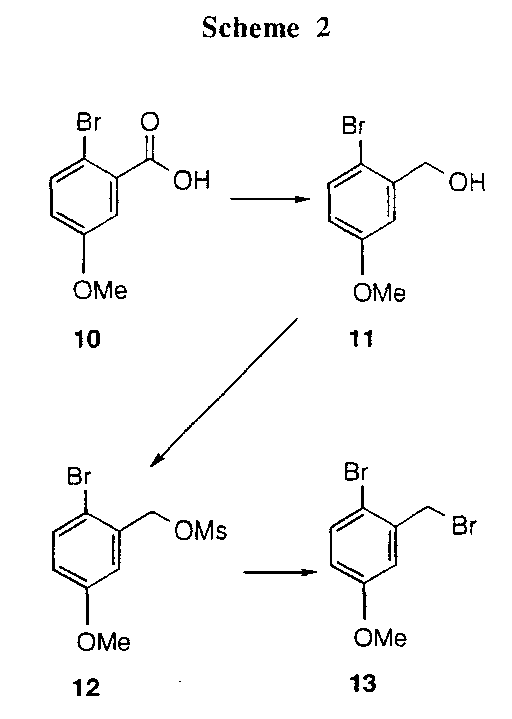

Commercial available 1,2-amino indanol is acylated (propionyl choride, K₂CO₃) to give amide **8**, which is then converted into the acetonide **9** (2-methoxypropene, pyridinium p-toluene-sulfonate (PPTS)). Acetonide **9** is then alkylated with the bromide **13**, (LiHMDS) to give **14**, which is then hydrolyzed (H⁺, MeOH) to give a mixture of acid and methyl ester **15**. Reduction (LAH) of the ester/acid mixture provided the alcohol **16** in high yield and optical purity. Protection of the alcohol **16** (TBSCI, imidazole) provided bromide **17**, the precursor to organolithium **17a**.

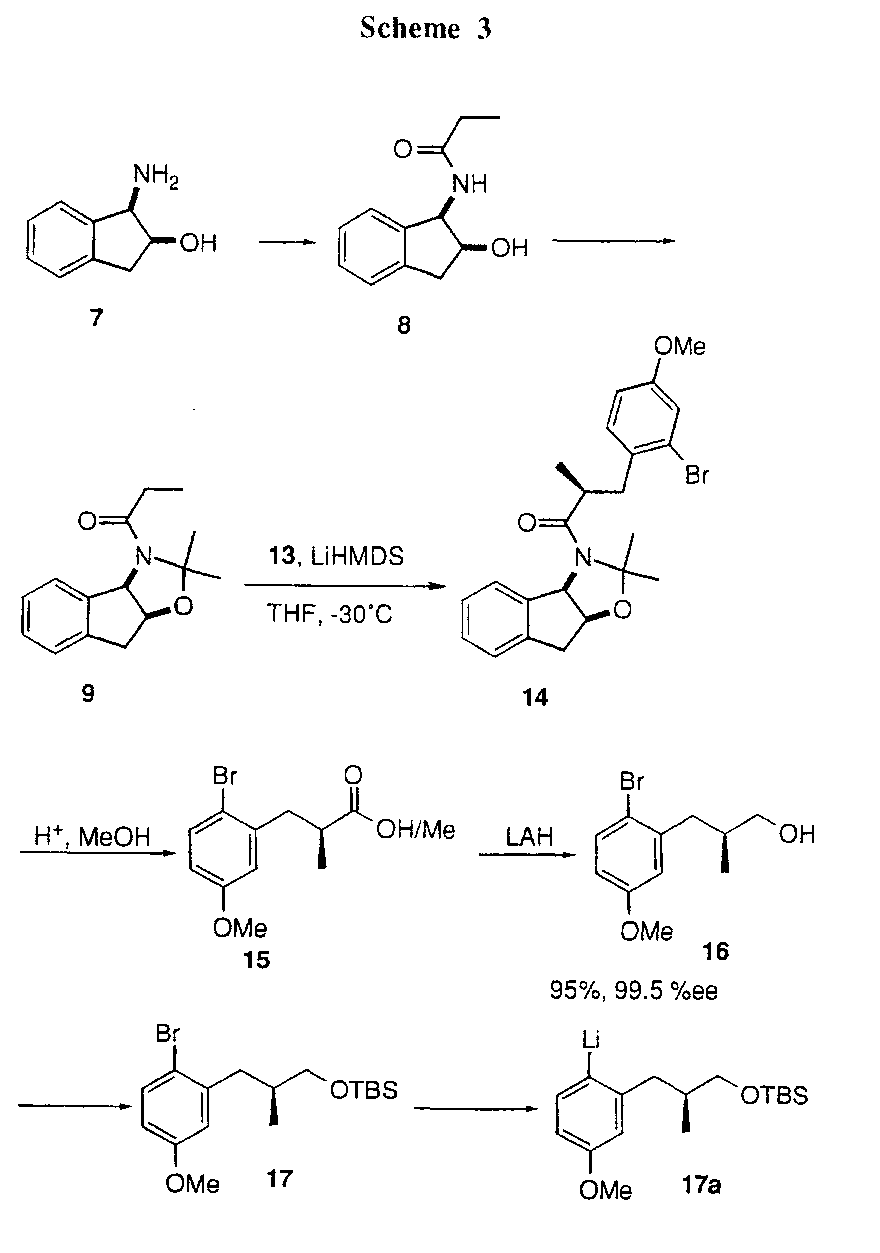

Compound **17a** is added to the α,β-unsaturated ester **5a** at -78° to -50°C. Work up with acetic acid, THF and water (to remove the auxiliary) affords compound **6a** in high yield and good selectivity. Addition of the Grignard leads to compound **18**. Oxidation with reagents such as NMO and TPAP with molecular sieves, followed by transesterification in n-butanol with Ti(OBu)₄ leads to compound **19** in good yield.

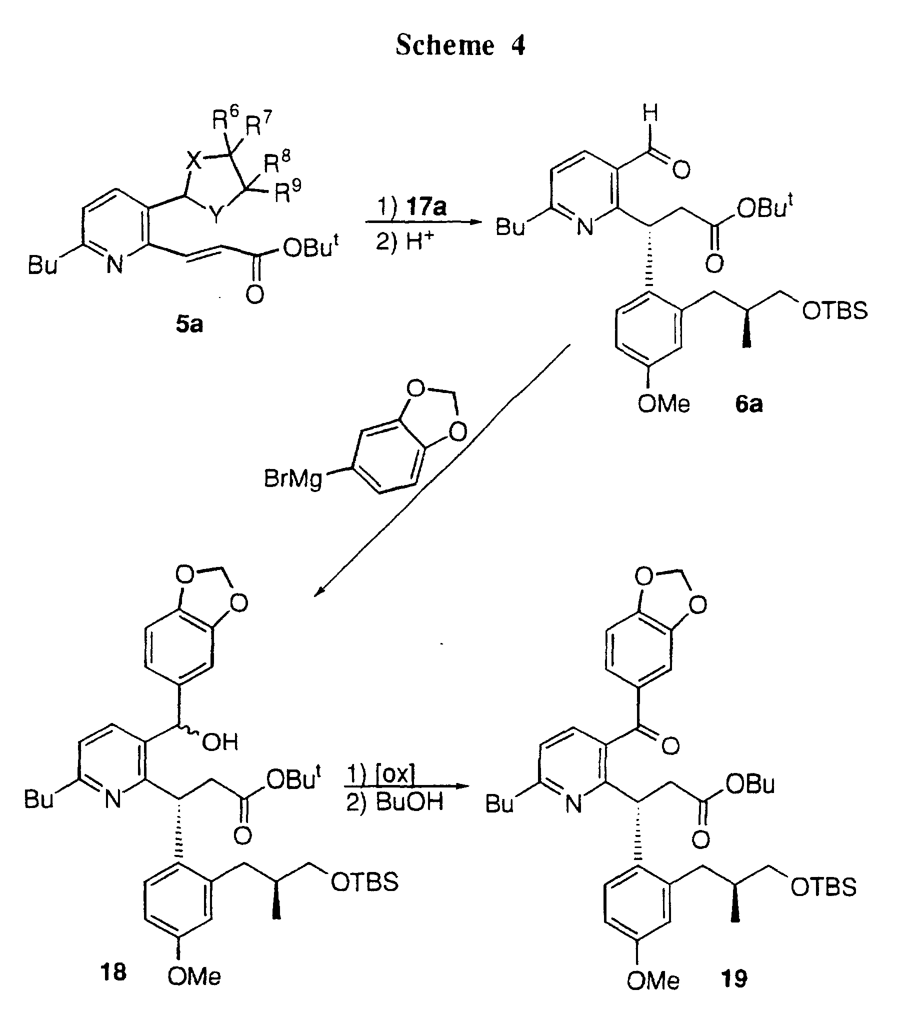

The instant invention can be understood further by the following examples, which do not constitute a limitation of the invention.

### EXAMPLE 1

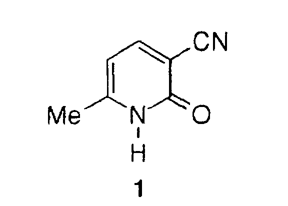

### Preparation of 1

Compound 1 is a commericially available starting material, for example, see Aldrich Chemical Company, Milwaukee, WI, USA 53201.

### EXAMPLE 2

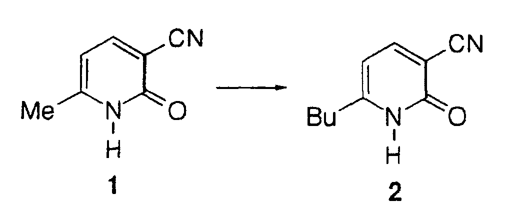

### Preparation of 2

Diisopropyl amine (MW 101.19, d 0.772, 2.1 equ, 20.54 mL) in 200 mL THF. Cool to -50°C and add n-BuLi (1.6 M in hexanes, 2.05 equ, 96 mL), allowing solution to warm to -20°C. Age 0-3°C for 15 min, then cool to -30°C and add **1** (MW 134.14, 75 mmol, 10.0 g). Age 0°C to 43°C for 2 h. Cool to -50°C and add bromopropane (MW 123.00, d 1.354, 1.0 equ, 6.8 mL). Warm to 25°C over 30 min, and age 30 min. Add NH₄Cl and CH₂Cl₂. Dry organic (magnesium sulfate) then evaporate *in vacuo* to afford 61% of **2**.

### EXAMPLE 3

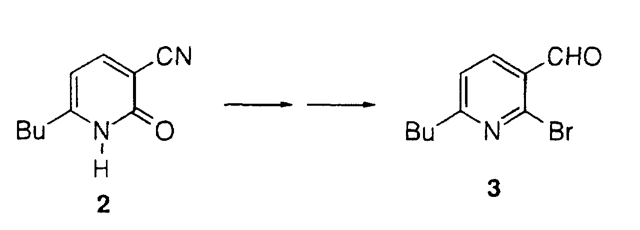

### Preparation of 3

Mix **2** (MW 176.22, 46 mmol) and PBr₃ (MW 270.70, d 2.880, 2.5 equ, 10.8 mL) and age at 160°C. After 2 h, cool to 25°C and add some CH₂Cl₂. Slowly quench by adding water. Separate layers and wash aqueous two times with CH₂Cl₂. Combine organic layers and dry (magnesium sulfate). Concentrate and isolate solid by silica gel chromatography (90:10 hexanes:ethyl acetate) in 60% yield (MW 239.12, 6.60 g).

Dissolve product of bromination reaction (MW 239.12, 27.6 mmol, 6.60 g) in 66 mL toluene and cool to -42°C. Slowly add DIBAL (1.5 M in toluene, 2 equ, 37 mL) and age 1 h at -42°C. Add HCl (2 N, 10 equ, 134 mL) and stir vigorously for 30 min. Dilute with ethyl acetate, separate layers, and wash aqueous with ethyl acetate. Combine organic layers, dry (magnesium sulfate), and concentrate *in vacuo* to afford 90% (MW 242.11, 6.01 g) of **3**.

### EXAMPLE 4a

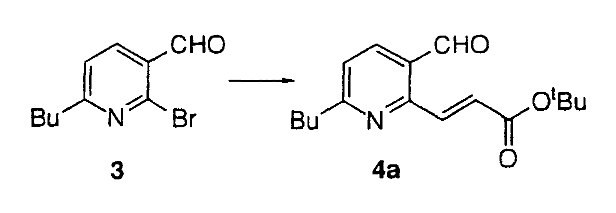

### Preparation of 4a

Dissolve **3** (MW 242.11, 24.8 mmol, 6.01 g) in 75 mL toluene. Add sodium acetate (MW 82, 3 equ, 6.13 g), t-butyl acrylate (MW 128.17, d 0.875, 2.5 equ, 9.08 mL), P(o-tolyl)3 (MW 304.38, 10 mol %, 755 mg) and allyl palladium chloride dimer (MW 365.85, 5 mol %, 455 mg). Age at reflux for 24 h. Cool, filter and evaporate *in vacuo*. Isolate **4a** (MW 289.37) by silica gel chromatography (92:8 hexanes:ethyl acetate) in 80% yield (5.74 g).

### EXAMPLE 4b

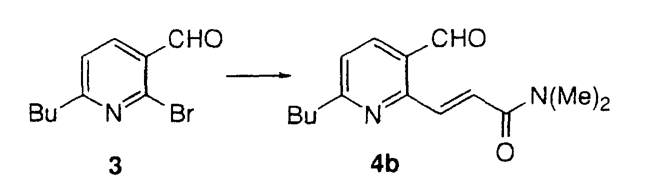

### Preparation of 4b

Dissolve **3** (MW 242.11, 24.8 mmol, 6.01 g) in 75 mL toluene. Add sodium acetate (MW 82, 3 equ, 6.13 g), dimethylacrylamide (MW 99.13, d 0.962, 1 equ, 2.55 mL), PPh3 (MW 262.29, 10 mol %, 653 mg) and allyl palladium chloride dimer (MW 365.85, 5 mol %, 455 mg). Age at 140°C in sealed tube for 24 h. Cool, filter and evaporate *in vacuo*. Isolate **4b** (MW 260.34) by silica gel chromatography (80:20 hexanes:ethyl acetate) in 70% yield (4.52 g).

### EXAMPLE 5a

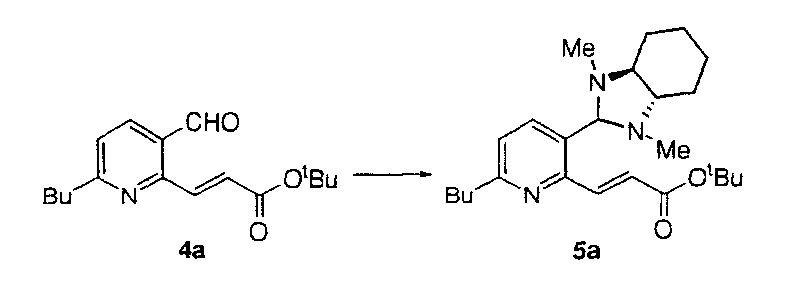

### Preparation of 5a

Dissolve **4a** (MW 289.37, 19.8 mmol, 5.74 g) in 53 mL CH₂Cl₂. Add (1R,2R)-N,N-dimethylcyclohexanediamine (MW 142.24, 1 equ, 2.83 g) and sieves (powdered, 1 wt equ, 5.74 g) and age 25°C for 8 h. Filter and concentrate filtrate *in vacuo* to afford **5a** (MW 413.60, 8.19 g) in quantitative yield.

### EXAMPLE 5b

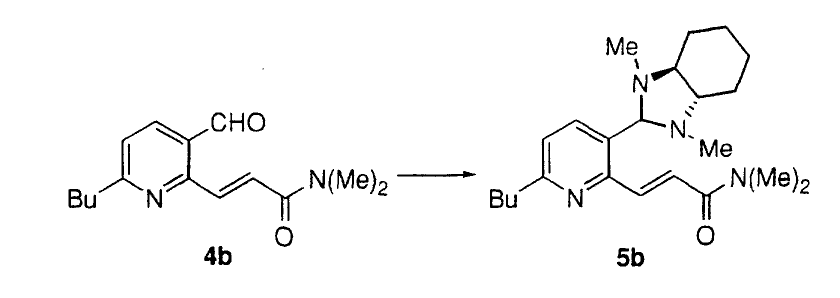

### Preparation of 5b

Dissolve **4b** (MW 260.34, 17.4 mmol, 4.53 g) in 40 mL CH₂Cl₂. Add (1R,2R)-N,N-dimethylcyclohexanediamine (MW 142.24, 1 equ, 2.47 g) and sieves (powdered, 1 wt equ, 4.53 g) and age 25°C for 8 h. Filter and concentrate filtrate *in vacuo* to afford **5b** (MW 384.57, 6.69 g) in quantitative yield.

### EXAMPLE 5c

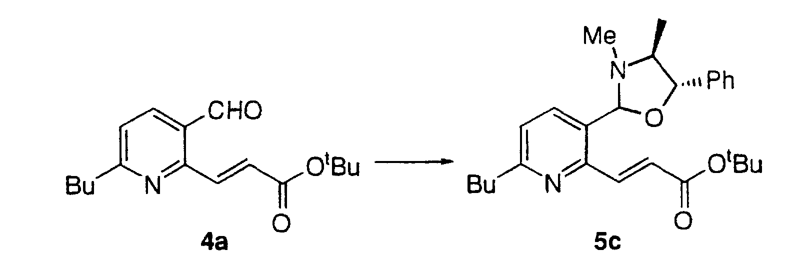

### Preparation of 5c

Dissolve **4a** (MW 289.37, 19.8 mmol, 5.74 g) in 53 mL toluene. Add (S,S)-pseudoephedrine (MW 165.24, 1.1 equ, 3.60 g) and 4 drops of concentrated HCl. Reflux with a Dean-Stark trap for 2h. Wash with saturated aqueous NaHCO₃ and extract with ethyl acetate. Dry organic layer with MgSO₄, then filter and concentrate filtrate *in vacuo* to afford **5c** (MW 4436.59, 8.64 g) in quantitative yield.
¹H NMR (CDCl₃) : 8.23 (d, *J*=11.78, 1 H), 7.88 (d, *J*=7.33, 1 H), 7.39 (m, 5 H), 7.16 (d, *J*=7.33, 1 H), 7.02 (d, *J*=11.78, 1 H), 5.31 (s, 1 H), 4.80 (d, *J*=9.18, 1 H), 2.80 (t, *J*=5.79, 2 H), 2.59 (m, 1 H), 2.19 (s, 3 H), 1.72 (m, 2 H), 1.56 (s, 9 H), 1.39 (m, 2 H), 1.27 (d, *J*=4.83, 3 H), 0.94 (t, *J*=6.76, 3 H).

### EXAMPLE 5d

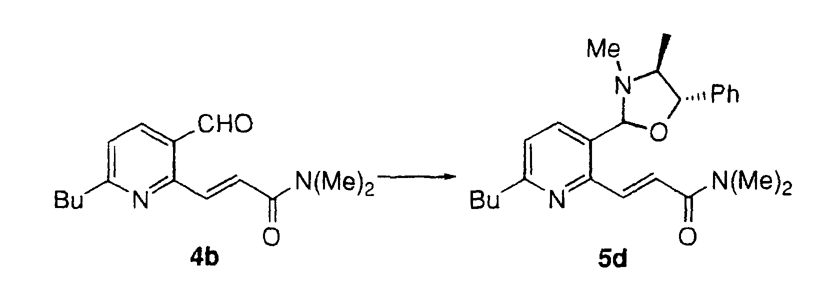

### Preparation of 5d

Dissolve **4b** (MW 260.34, 117.4 mmol, 5.74 g) in 53 mL toluene. Add (S,S)-pseudoephedrine (MW 165.24, 1.1 equ, 3.16 g) and 4 drops of concentrated HCl. Reflux with a Dean-Stark trap for 2h. Wash with saturated aqueous NaHCO₃ and extract with ethyl acetate. Dry organic layer with MgSO₄, then filter and concentrate filtrate *in vacuo* to afford **5c**.

### EXAMPLE 6a

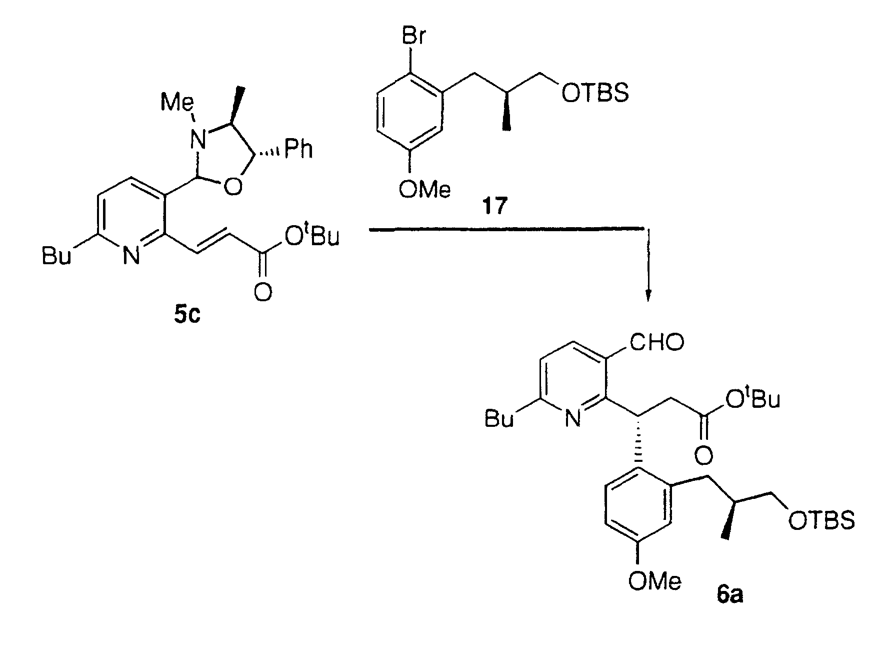

### Preparation of 6a

Dissolve **17** (see Example 17, MW 373.41, 2 equ, 14.79 g) in 85 mL THF. Cool to -78°C and add t-BuLi (1.7 M in pentane, 4 equ, 46.6 mL), maintaining temperature below -70°C. Age 15 min, then slowly add solution of **5c** (MW 436.59, 19.8 mmol, 8.64 g) in 65 mL THF. Age 1 h at -78°C, then cannula into cold aq NH₄Cl (100 mL). Add ethyl acetate and separate layers. Wash aqueous with ethyl acetate. Combine organic layers and wash with brine, then dry (magnesium sulfate) and evaporate *in vacuo*. ¹H NMR provides de data. Add THF (75 mL), acetic acid (AcOH) (30 mL) and water (10 mL). Age 5 h at 25°C. Separate layers and wash aqueous two times with ethyl acetate. Combine organic layers, wash with brine, dry (magnesium sulfate), and evaporate *in vacuo*. **6a** (MW 583.89) is isolated in 85% yield (9.83 g) by silica gel chromatography (92:8 hexanes:ethyl acetate).
¹H NMR (C₆D₆) : 10.5 (s, 1 H), 7.72 (d, *J*=7.85, 1 H), 7.30 (d, *J*=8.64, 1 H), 6.83 (d, *J*=8.05, 1 H), 6.59 (dd, *J*=8.65, 2.61, 1 H), 6.56 (d, *J*=7.99, 1 H), 5.92 (m, 1 H), 3.85 (dd, *J*=16.32, 10.77, 1 H), 3.48 (m, 2 H), 3.32 (s, 3 H), 3.01 (dd, *J*=14.11, 6.77, 1 H), 2.87 (dd, *J*=16.30, 3.91, 1 H), 2.79 (dd, *J*=13.25, 6.21, 1 H), 2.68 (t, *J*=7.66, 2 H), 2.10 (m, 1 H), 1.72 (m, 2 H), 1.30 (s, 9 H), 1.25 (m, 2 H), 1.01 (s, 9 H), 0.95 (d, *J*=6.42, 3 H), 0.94 (t, *J*=8.40, 3 H), 0.10 (d, *J*=5.83, 6 H).

### EXAMPLE 6b

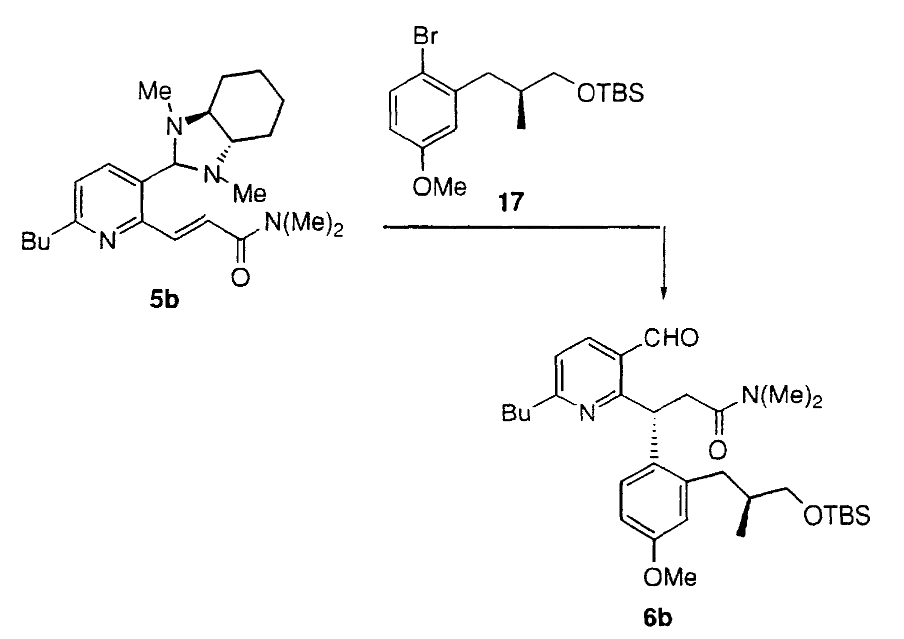

### Preparation of 6b

Dissolve **17** (see Example 17, MW 373.41, 2 equ, 12.99 g) in 70 mL THF. Cool to -78°C and add t-BuLi (1.7 M in pentane, 4 equ, 40.9 mL), maintaining temperature below -70°C. Age 15 min, then slowly add solution of **5b** (MW 384.57, 17.4 mmol, 6.69 g) in 55 mL THF. Age 1 h at -78°C, then cannula into cold aq NH₄Cl (100 mL). Add ethyl acetate and separate layers. Wash aqueous with ethyl acetate. Combine organic layers and wash with brine, then dry (magnesium sulfate) and evaporate *in vacuo*. ¹H NMR provides de data. Add THF (55 mL), AcOH (20 mL) and water (8 mL). Age 5 h at 25°C. Separate layers and wash aqueous two times with ethyl acetate. Combine organic layers, wash with brine, dry (magnesium sulfate), and evaporate *in vacuo*. **6b** (MW 678.99) is isolated in 75% yield (8.86 g) by silica gel chromatography (70:30 hexanes:ethyl acetate).
¹H NMR (CDCl₃) : 10.30 (s, 1 H), 7.99 (d, *J*=4.74, 1 H), 7.11 (d, *J*=3.19, 1 H), 6.89 (d, *J*=8.61, 1 H), 6.78 (d, *J*=2.76, 1 H), 6.59 (t, *J*=2.78, 1 H), 5.70 (t, *J*=2.86, 1 H), 3.87 (dd, *J*=11.18, 4.29, 1 H), 3.74 (s, 3 H), 3.58 (m, 2 H), 3.11 (s, 3 H), 3.25 (dd, *J*=14.35, 6.25, 1 H), 2.88 (s, 3 H), 2.84 (m, 2H), 2.68 (dd, *J*=14.35, 8.30, 1 H), 2.47 (dd, *J*=9.02, 2.89, 1 H), 2.09 (m, 1 H), 1.75 (m, 2 H), 1.39 (m, 2 H), 0.99 (t, *J*=3.49, 3 H), 0.92 (s, 9 H), 0.92 (d, *J*=7.15, 6 H), 0.08 (d, *J*=1.91, 6 H).
¹³C NMR (CDCl₃) : 190.5, 171.6, 165.9, 163.7, 157.9, 139.3, 137.2, 135.5, 130.0, 127.1, 120.8, 115.5, 111.7, 67.8, 55.11, 39.7, 38.9, 38.4, 37.2, 36.8, 36.0, 35.4, 26.0 (3 C), 22.3, 18.4, 17.3, 14.7, -5.3 (2 C).

### EXAMPLE 7

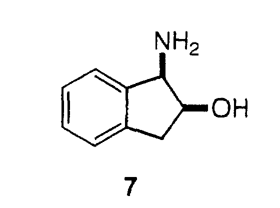

### Preparation of 7

Compound 7 is a commericially available starting material, for example, see DSM Andeno, Grubbenvorsterweg 8, P.O. Box 81, 5900 AB Venlo,The Netherlands.

### EXAMPLE 8

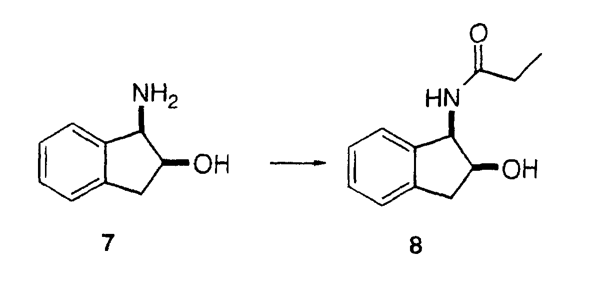

### Preparation of 8

Na₂CO₃ (MW 105.99, 1.5 equ, 8.8 g) dissolved in 82 mL water. Add a solution of (1R,2S) amino indanol **7** (MW 149.19, 55.0 mmol, 8.2 g) in 160 mL CH₂Cl₂. Cool to -5°C and add propionyl chloride (MW 92.53, d 1.065, 1.3 equ, 6.2 mL). Warm to 25°C and age 1 h. Separate layers and dry organic (magnesium sulfate). Concentrate *in vacuo* to afford **8** (MW 205.26, 10 g) in 89% isolated yield.

### EXAMPLE 9

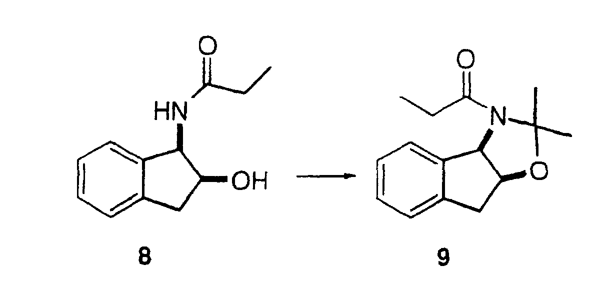

### Preparation of 9

To a solution of **8** (MW 205.26, 49.3 mmol, 10 g) in 200 mL THF, add pyridinium *p*-toluenesulfonate (PPTS) (MW 251.31, 0.16 equ, 2g) then methoxypropene (MW 72.11, d 0.753, 2.2 equ, 10.4 mL). Age 2 h at 38°C, then add aqueous sodium bicarbonate and ethyl acetate. The organic layer was dried (magnesium sulfate). After concentration *in vacuo*, **9** (MW 245.32, 12.09 g) was formed in quantitative yield.

### EXAMPLE 10

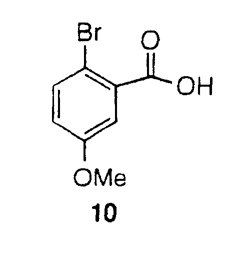

### Preparation of 10

Compound 10 is a commericially available starting material, for example, see Lancaster Synthesis, P.O. Box 1000, Windham, NH 03087-9977 or Ryan Scientific, Inc., P.O. Box 845, Isle of Palms, SC 29451-0845.

### EXAMPLE 11

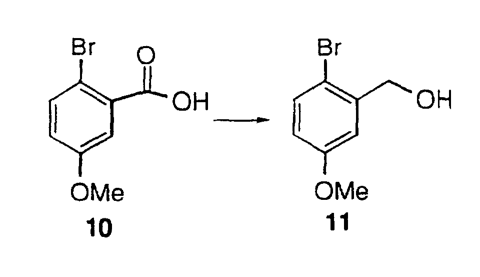

### Preparation of 11

**10** (MW 231.05, 130 mmol, 30.0 g) in 300 mL CH₂Cl₂ at 0°C. Add BH₃-SMe₂ (3 equ, 25.2 mL) and age for 2 h at 25°C. Quench into aqueous 2 N HCI and separate layers. Dry organic (magnesium sulfate) and concentrate *in vacuo* to obtain 94% yield of **11** (MW 217.06, 25.5 g).

### EXAMPLE 12

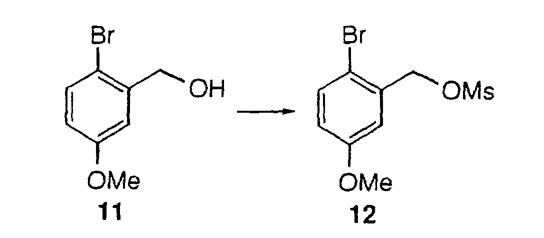

### Preparation of 12

Dissolve **11** (MW 217.06, 47.2 mmol, 10.24 g) in 55 mL CH₂Cl₂ and cool to -20°C. Add DIEA (MW 129.25, d 0.742, 1.3 equ, 10.69 mL) then methane sulfonyl chloride (MsCl) (MW 114.55, d 1.480, 1.2 equ, 4.38 mL). Age -5°C to 0°C for 1 h then quench into 55 mL water. Extract with CH₂Cl₂ then wash with 1N H₂SO₄ (40 mL), then brine. Dry organic layers (magnesium sulfate) and concentrate *in vacuo* to afford **12** (MW 295.15, 13.23 g) in 95% yield.

### EXAMPLE 13

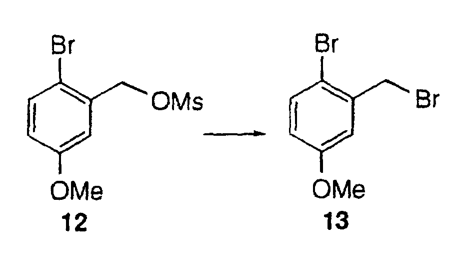

### Preparation of 13

**12** (MW 295.15, 44.8 mmol, 13.23 g) in 44 mL dimethylacetamide (DMAC). Add NaBr (MW 102.90, 2 equ, 9.22 g) and age 1h. Add 88 mL water and collect solid by filtration. Wash cake with water and dry by suction. Quantitative yield of **13** (MW 279.96, 12.54 g) is obtained.

### EXAMPLE 14

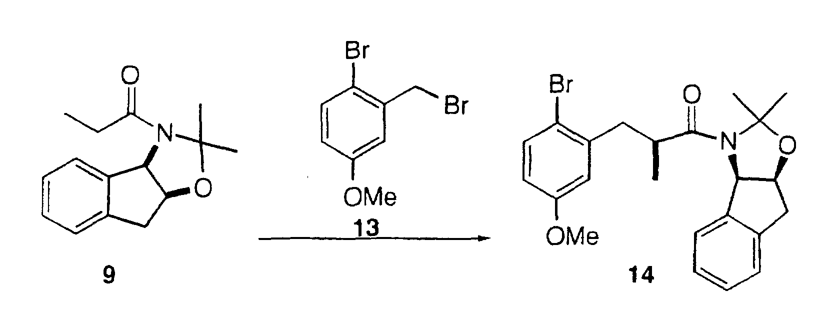

### Preparation of 14

**9** (MW 245.32, 1.1 equ, 89.1 g) in 1 L THF, cooled to -50°C. Add LiHMDS (1.0 M in THF, 1.5 equ, 545 mL) and age 1.5 h, warming to -30°C. Add **13** (MW 279.96, 327 mmol, 91.3 g) in 300 mL THF, and age -35°C for 1 h. Warm to -10°C over 1 h, then quench into aqueous NH₄Cl. Separate layers and extract with ethyl acetate. Dry organic and concentrate *in vacuo* to afford crude **14** (MW 444.37).

### EXAMPLE 15

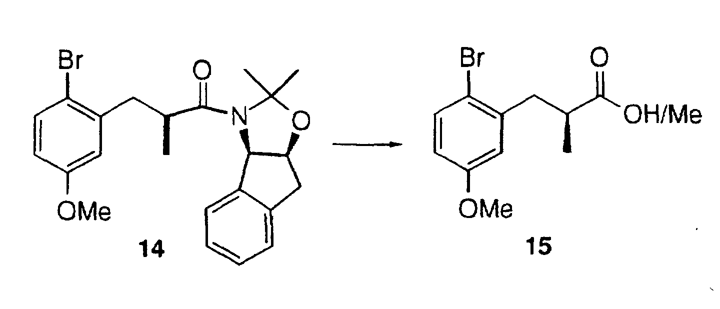

### Preparation of 15

**14** in 1 L MeOH and cooled to 10°C. Bubble in HCl gas for 1 h until reaction is complete. 2 L H₂O added and the product was filtered. The cake was washed with H₂O and dried to give the product hydroxyamide, which was then dissolved in 1 L MeOH and 1.5 L 6N HCI and refluxed overnight. The mixture was cooled to 25°C and extracted with CH₂Cl₂ to give, after concentration, compounds **15** (60 g, 64% from bromide **13**).

### EXAMPLE 16

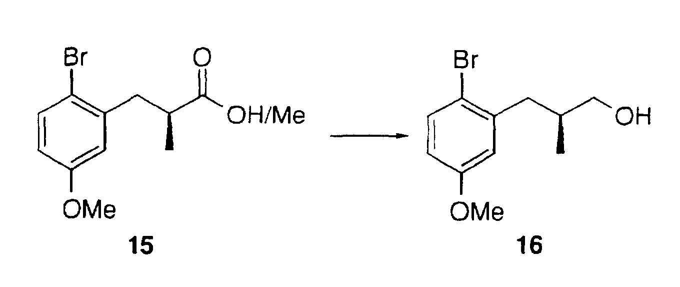

### Preparation of 16

**15** (mixture of acid and ester, 26.88 mmol) in 150 mL THF at -78°C. Add lithium aluminum hydride (LiAlH₄) (1 M in THF, 2 equ, 53.76 mL) over 30 min. Warm to 25°C over 1 h, then quench into aqueous NH₄Cl. Add ethyl acetate, extract ethyl acetate. Wash organics with brine, dry (magnesium sulfate), and concentrate *in vacuo* to afford 95% yield of **16** (MW 259.14, 6.62 g).

### EXAMPLE 17

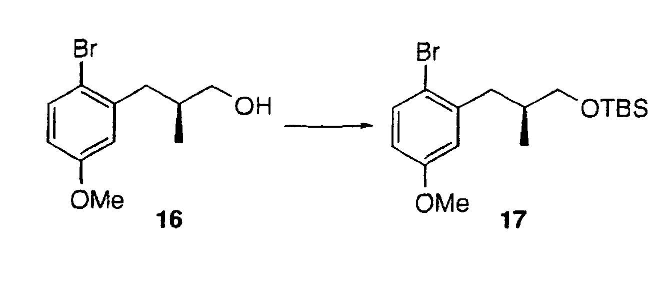

### Preparation of 17

**16** (MW 259.14, 25.54 mmol, 6.62 g) in 35 mL CH₂Cl₂ and cool to 0°C. Add imidazole (MW 68.08, 2.5 equ, 4.35 g) and then tert-butyldimethylsilyl chloride (TBSCl) (MW 150.73, 1 equ, 3.85 g). Age 1 h at 25°C then quench with aqueous NaHCO₃ and add ethyl acetate. Extract with ethyl acetate, then dry organic layer (magnesium sulfate) and concentrate *in vacuo* to afford a quantitative yield of **17** (MW 373.41, 9.54 g).
¹H NMR (CDCl₃) : 7.41 (d, *J*=8.74, 1H), 6.77 (d, *J*=3.04, 1H), 6.63 (dd, *J*=8.73, 3.06, 1H), 3.78 (s, 3 H), 3.50 (d, *J*=5.75, 2 H), 2.89 (dd, *J*=13.31, 6.15, 1 H), 2.45 (dd, *J*=13.30, 8.26, 1 H), 2.03 (m, 1 H), 0.94 (s, 9 H), 0.92 (d, *J*=5.01, 3 H), 0.07 (s, 6 H).
¹³C NMR (CDCl₃) : 159.1, 141.6, 133.2, 117.0, 115.4, 113.2, 67.4, 55.4, 39.7, 36.3, 26.0 (3C), 18.4, 16.5, -5.3 (2C).

### EXAMPLE 18

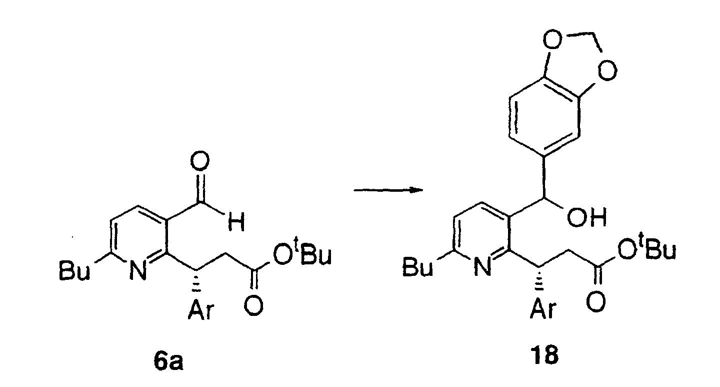

Ar represents:

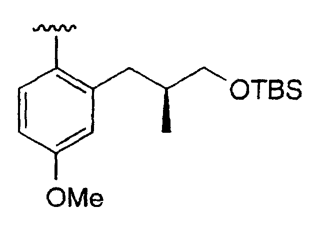

### Preparation of 18

Prepare 0.5 M Grignard solution from 4-bromo-1,2-(methylenedioxy)benzene (MW 201.01, 42.1 mmol, 8.46 g) and Mg (MW 24.31, 1.5 equ, 1.54 g) in 84 mL THF. Dissolve **6a** (MW 583.89, 16.8 mmol, 9.83 g) in 80 mL THF and cool to -78°C. Slowly add Grignard solution (2.5 equ, 0.5 M, 84 mL) and age 30 min. Quench into aqueous NH₄Cl and add ethyl acetate. Wash organic with brine, dry (magnesium sulfate) and evaporate *in vacuo*. Carry crude into oxidation.

### EXAMPLE 19

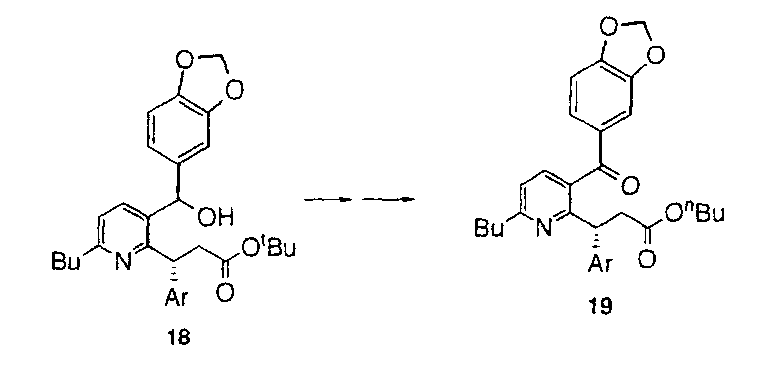

### Preparation of 19

Crude **18** (MW 706.01, 16.8 mmol) in 150 mL ACN. Add NMO (MW 117.15, 3 equ, 5.90 g), sieves (powdered, 3 wt equ, 35.6 g), and TPAP (MW 351.43, 10 mol %, 590 mg) and age 25°C for 2 h. Concentrate to remove ACN, then elute through silica gel pad with ethyl acetate. Concentrate *in vacuo*, then chromatograph (90:10 hexanes:ethyl acetate) to isolate the oxidation product (85% yield over two steps).

Dissolve in 100 mL n-BuOH and add Ti(OBu)₄ (MW 340.366, 5 equ, 28.59 g), Reflux for 48 h, then quench into water and add ethyl acetate. Filter through celite, separate the layers, and wash the organic with brine. Dry (magnesium sulfate) and evaporate *in vacuo* to afford 81% yield (over three steps) of **19** (MW 703.99, 9.58 g).

### EXAMPLES 20-26

Following the procedures described in Examples 6a and 6b, the nucleophiles were added to the acceptors listed below and the diastereomeric ratios (%de) of the products were determined by evaluation of the ¹H NMR data and are shown below.

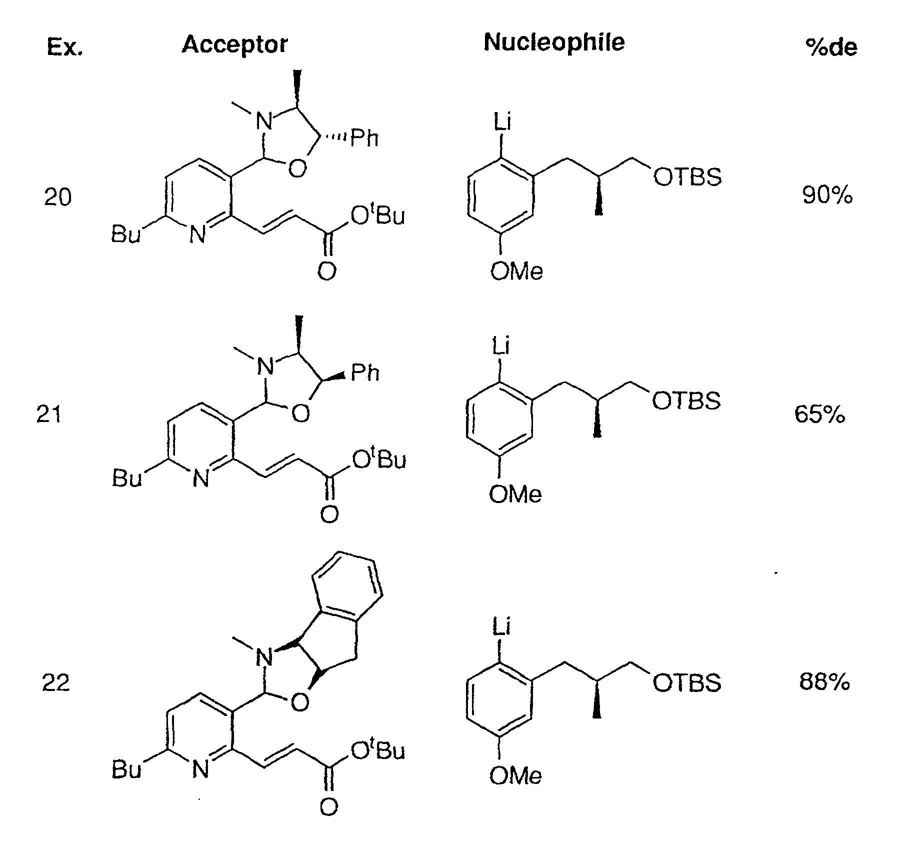

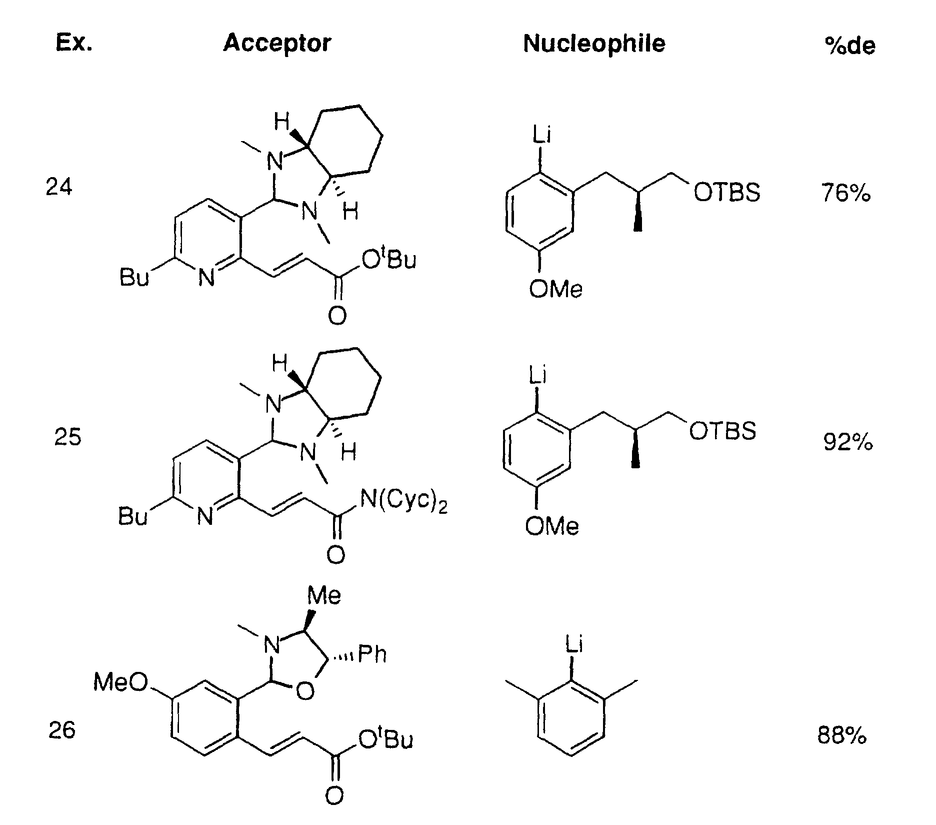

## Claims

1. A compound of Formula I: 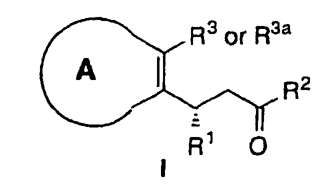 wherein 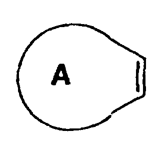 represents:
a) 5- or 6-membered heterocyclyl containing one, two or three double bonds, but at least one double bond and 1, 2 or 3 heteroatoms selected from O, N and S, the heterocyclyl is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
b) 5- or 6-membered carbocyclyl containing one or two double bonds, but at least one double bond, the carbocyclyl is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
c) aryl, wherein aryl is as defined below,
C₁-C₈ alkoxy, C₁-C₈ alkyl, C₂-C₈ alkenyl, C₂-C₈ alkynyl, or C₃-C₈ cycloalkyl, are unsubstituted or substituted with one, two or three substituents selected from the group consisting of: OH, CO₂R⁴, Br, Cl, F, I, CF₃, N(R⁵)₂, C₁-C₈ alkoxy, C₃-C₈ cycloalkyl, CO(CH₂)ₙCH₃, and CO(CH₂)ₙCH₂N(R⁵)₂,
n is 0 to 5;
R¹ is:
a) C₁-C₈ alkyl, C₂-C₈ alkenyl, C₂-C₈ alkynyl, C₃-C₈ cycloalkyl,
b) aryl, or
c) heteroaryl;
aryl is defined as phenyl or naphthyl, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, CO(CH₂)ₙCH₂N(R⁵)₂, and when two substituents are located on adjacent carbons they can join to form a 5- or 6-membered ring with one, two or three heteroatoms selected from O, N, and S, which is unsubstituted or substituted with one, two or three substituents selected from the group consisting of: H, OH, CO₂R⁶, Br, Cl, F, I, CF₃, N(R⁷)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
heteroaryl is defined as a 5- or 6-membered aromatic ring containing 1, 2 or 3 heteroatoms selected from O, N and S, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, and CO(CH₂)ₙCH₂N(R⁵)₂,
R² is OR⁴ or N(R⁵)₂;
R³ is 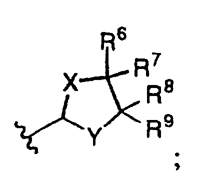
R^{3a} is:
a) CHO,
b) -CO-C₁-C₈ alkyl,
c) -CO-aryl, or
d) -CO-heteroaryl;
X and Y are independently: O, S, or NR⁵;
R⁴ is branched C₃-C₈ alkyl;
R⁵ is: C₁-C₈ alkyl, or aryl; and
R⁶, R⁷, R⁸ and R⁹ are independently: H, C₁-C₈ alkyl, and aryl, such that either R⁶ and R⁷ are not the same and/or R⁸ and R⁹ are not the same, or R⁶ and R⁸ or R⁷ and R⁹ can join to form a 5- or 6-membered ring, which is unsubstituted or substituted with one, two or three substituents selected from the group consisting of: OH, CO₂R⁴, Br, Cl, F, I, CF₃, N(R⁵)₂, C₁-C₈ alkoxy, C₁-C₈ alkyl, C₂-C₈ alkenyl, C₂-C₈ alkynyl, or C₃-C₈ cycloalkyl, CO(CH₂)ₙCH₃, CO(CH₂)ₙCH₂N(R⁵)₂.

2. A process for the preparation of a compound of formula I: 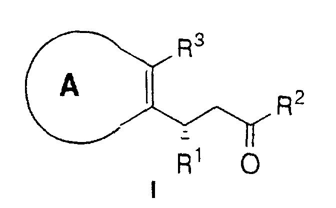 wherein the compound of formula I is as defined in Claim 1, comprising reacting a α,β-unsaturated ester or amide 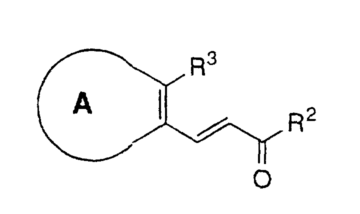 with an organolithium compound, R¹Li, in the presence of an aprotic solvent at a temperature range of about -78°C to about 0°C.

3. The process as recited in Claim 2, wherein the number of equivalents of the organolithium compound, R¹Li, is 1 to about 4.

4. The process as recited in Claim 3, wherein the aprotic solvent is selected from the group consisting of tetrahydrofuran, diethyl ether, methyl t-butyl ether, benzene, toluene, hexane, pentane, dioxane and a mixture of said solvents.

5. The process as recited in Claim 4, wherein the temperature range is about -78°C to about -20°C.

6. A process for the preparation of a compound of formula I: 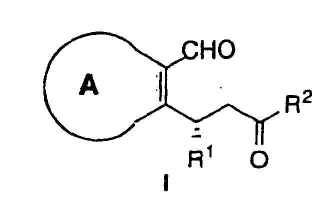 wherein the compound of formula I is as defined in Claim 1, comprising the steps of:
1) reacting an α,β-unsaturated ester or amide 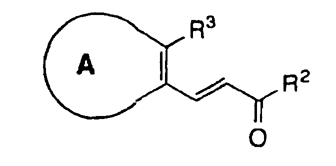 with an organolithium compound, R¹Li, in the presence of an aprotic solvent at a temperature range of about -78°C to about 0°C to give the conjugate adduct; and
2) removing the chiral auxiliary, R³, with aqueous acid and tetrahydrofuran to give the compound of Formula I.

7. The process as recited in Claim 6, wherein the number of equivalents of the organolithium compound, R¹Li, is I to about 4.

8. The process as recited in Claim 7, wherein the aprotic solvent is selected from the group consisting of tetrahydrofuran, diethyl ether, methyl t-butyl ether, benzene, toluene, hexane, pentane, dioxane and a mixture of said solvents.

9. The process as recited in Claim 8, wherein the temperature range is about -78°C to about -20°C.

10. The process as recited in Claim 9, wherein the aqueous acid is aqueous acetic acid.

11. A process for the preparation of 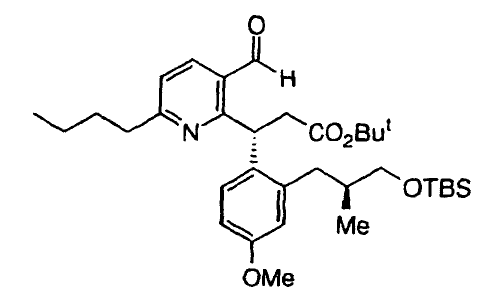 comprising reacting a α,β-unsaturated ester 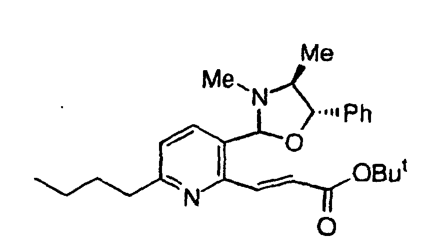 with an organolithium compound 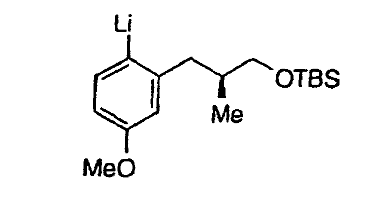 in the presence of an aprotic solvent at a temperature range of about -78°C to about -20°C.

12. The process as recited in Claim 11, wherein the number of equivalents of the organolithium compound, R¹Li, is I to about 4.

13. The process as recited in Claim 12, wherein the aprotic solvent is selected from the group consisting of tetrahydrofuran, diethyl ether, methyl t-butyl ether, benzene, toluene, pentane, hexane, dioxane and a mixture of said solvents.

14. The process as recited in Claim 13, wherein the temperature range is about -78°C to about -50°C.

15. The process as recited in Claim 14, wherein the number of equivalents of the organolithium compound, R¹Li, is 1.5 to about 2.5.

16. The process as recited in Claim 15, wherein the aprotic solvent is tetrahydrofuran.

17. The process as recited in Claim 16, wherein the temperature range is about -78°C to about -70°C.

18. A process for the preparation of a ketone 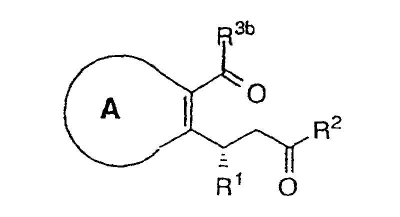 wherein 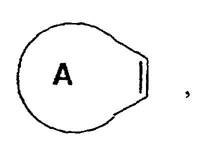 R¹ and R² are as defined in Claim 1; and
R^{3b} is:
a) C₁-C₈ alkyl,
b) aryl, or
c) heteroaryl;
wherein aryl is as defined in Claim 1;
comprising the steps of:
1) reacting a α,β-unsaturated ester or amide 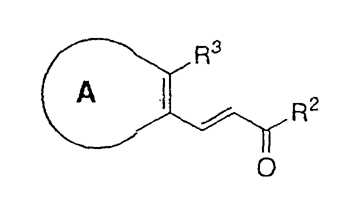 with an organolithium compound, R¹Li, in the presence of an aprotic solvent at a temperature range of about -78°C to about 0°C to give a conjugate adduct 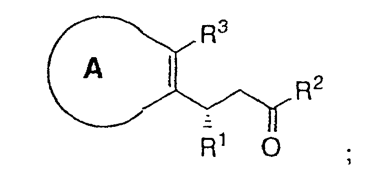
2) removing the chiral auxiliary with aqueous acid and tetrahydrofuran to give the aldehyde 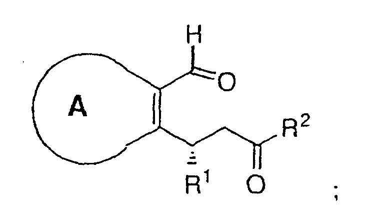
3) reacting the aldehyde with a Grignard reagent or organolithium reagent formed with R^{3b}Z, where Z is Br, Cl, or I to form an alcohol 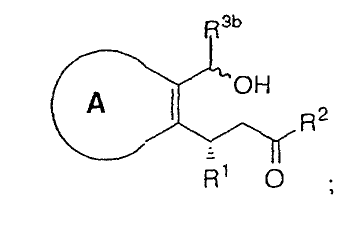
4) oxidizing the alcohol formed with an oxidizing agent to give the ketone

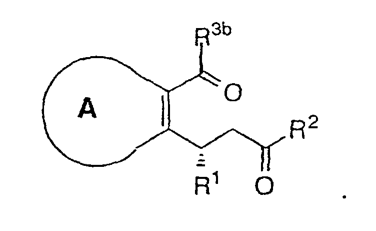

19. The process as recited in Claim 18, wherein the number of equivalents of the organolithium compound in the first step is 1 to about 4.

20. The process as recited in Claim 19, wherein the aprotic solvent in the first step is selected from the group consisting of tetrahydrofuran, diethyl ether, methyl t-butyl ether, benzene, toluene, pentane, hexane, dioxane and a mixture of said solvents.

21. The process as recited in Claim 20, wherein the temperature range in the first step is about -78°C to about -50°C.

22. The process as recited in Claim 21, wherein the aqueous acid in the second step is aqueous acetic acid.

23. The process as recited in Claim 22, wherein the oxidizing agent in the fourth step is 4-methylmorpholine-N-oxide and tetrapropylammonium perruthenate(VII).

24. A process for the preparation of a ketone of formula: 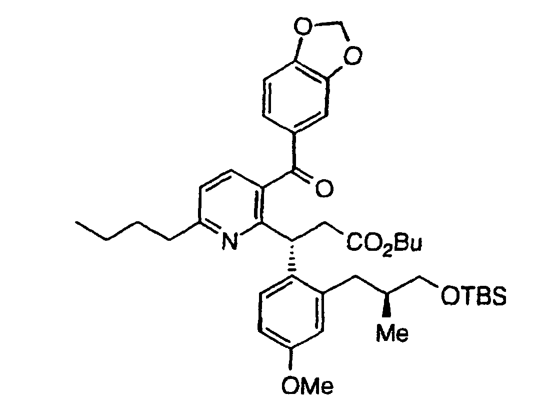 comprising the steps of:
1) reacting a α,β-unsaturated ester 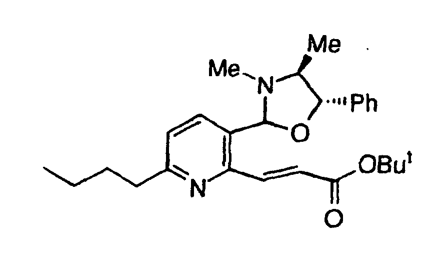 with an organolithium compound 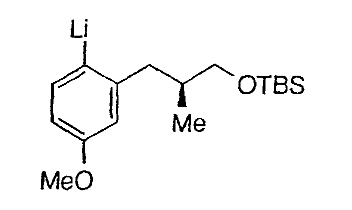 in the presence of an aprotic solvent at a temperature range of about -78°C to about -20°C to give a conjugate adduct 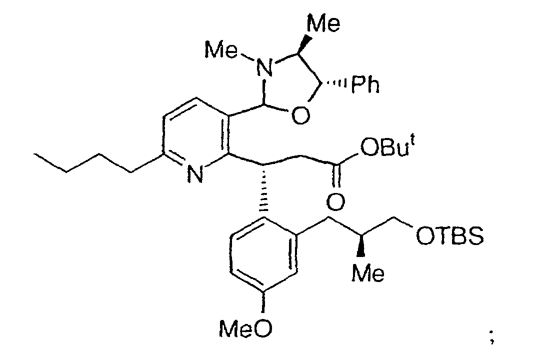
2) removing the chiral auxiliary with aqueous acid and tetrahydrofuran to give the aldehyde 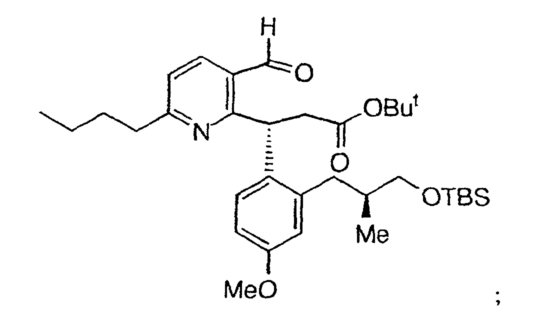
3) reacting the aldehyde with a Grignard reagent or organolithium reagent formed with 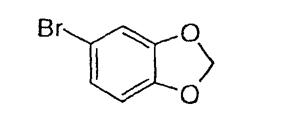 to form the alcohol 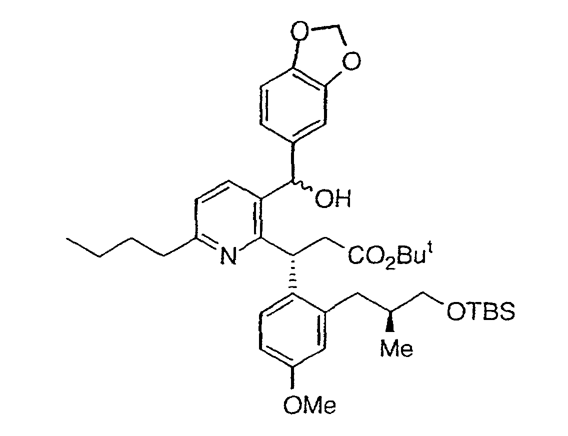
4) oxidizing the alcohol formed with an oxidizing agent to give a ketone 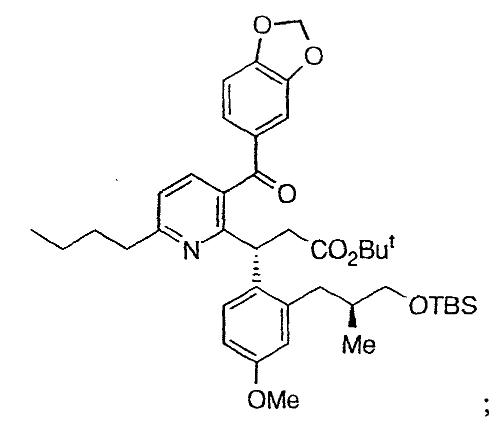 and
5) transesterifying the ester with n-butanol and a Lewis acid to give the desired ketone.

25. The process as recited in Claim 24, wherein the number of equivalents of the organolithium compound in the first step is 1 to about 4.

26. The process as recited in Claim 25, wherein the aprotic solvent in the first step is selected from the group consisting of tetrahydrofuran, diethyl ether, methyl t-butyl ether, benzene, toluene, pentane, hexane, dioxane and a mixture of said solvents.

27. The process as recited in Claim 26, wherein the temperature range in the first step is about -78°C to about -50°C.

28. The process as recited in Claim 27, wherein the aqueous acid in the second step is aqueous acetic acid.

29. The process as recited in Claim 28, wherein the Grignard reagent in the third step is

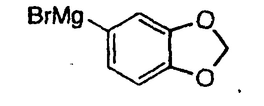

30. The process as recited in Claim 28, wherein the organolithium reagent in the third step is

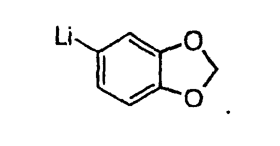

31. The process as recited in Claim 29, wherein the oxidizing agent in the fourth step is 4-methylmorpholine-N-oxide and tetrapropylammonium perruthenate(VII).

32. The process as recited in Claim 31, wherein the fifth step is conducted in the presence of a Lewis acid selected from the group consisting of: Ti(OEt)₄, Ti(OiPr)₄, and Ti(OBu)₄.

## Patentansprüche

1. Eine Verbindung der Formel I: 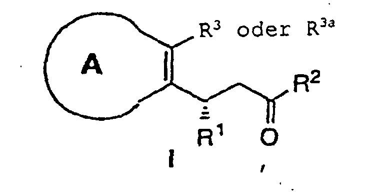 wobei 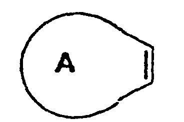 bedeutet:
a) 5- oder 6gliedriges Heterocyclyl mit einer, zwei oder drei Doppelbindungen, jedoch wenigstens einer Doppelbindung, und 1, 2 oder 3 Heteroatomen, ausgewählt aus O, N und S, wobei das Heterocyclyl unsubstituiert oder substituiert ist mit einem, zwei oder drei Substituenten, ausgewählt aus der Gruppe, bestehend aus: OH, CO₂R⁴, Br, Cl, F, I, CF₃, N(R⁵)₂, C₁-C₈-Alkoxy, C₁-C₈-Alkyl, C₂-C₈-Alkenyl, C₂-C₈-Alkinyl oder C₃-C₈-Cycloalkyl, CO(CH₂)ₙCH₃ und CO(CH₂)ₙCH₂N(R⁵)₂,
b) 5- oder 6gliedriges Carbocyclyl mit einer oder zwei Doppelbindungen, jedoch wenigstens einer Doppelbindung, wobei das Carbocyclyl unsubstituiert oder substituiert ist mit einem, zwei oder drei Substituenten, ausgewählt aus der Gruppe, bestehend aus: OH, CO₂R⁴, Br, Cl, F, I, CF₃, N(R⁵)₂, C₁-C₈-Alkoxy, C₁-C₈-Alkyl, C₂-C₈-Alkenyl, C₂-C₈-Alkinyl oder C₃-C₈-Cycloalkyl, CO(CH₂)ₙCH₃ und CO(CH₂)ₙCH₂N(R⁵)₂,
c) Aryl, wobei Aryl wie nachstehend definiert ist,
C₁-C₈-Alkoxy, C₁-C₈-Alkyl, C₂-C₈-Alkenyl, C₂-C₈-Alkinyl oder C₃-C₈-Cycloalkyl unsubstituiert oder substituiert sind mit einem, zwei oder drei Substituenten, ausgewählt aus der Gruppe, bestehend aus: OH, CO₂R⁴, Br, Cl, F, I, CF₃, N(R⁵)₂, C₁-C₈-Alkoxy, C₃-C₈-Cycloalkyl, CO(CH₂)ₙCH₃ und CO(CH₂)ₙCH₂N(R⁵)₂,
n 0 bis 5 ist,
R¹ ist:
a) C₁-C₈-Alkyl, C₂-C₈-Alkenyl, C₂-C₈-Alkinyl, C₃-C₈-Cycloalkyl,
b) Aryl oder
c) Heteroaryl,
Aryl als Phenyl oder Naphthyl definiert ist, das unsubstituiert oder substituiert ist mit einem, zwei oder drei Substituenten, ausgewählt aus der Gruppe, bestehend aus: OH, CO₂R⁴, Br, Cl, F, I, CF₃, N(R⁵)₂, C₁-C₈-Alkoxy, C₁-C₈-Alkyl, C₂-C₈-Alkenyl, C₂-C₈-Alkinyl oder C₃-C₈-Cycloalkyl, CO(CH₂)ₙCH₃, CO(CH₂)ₙCH₂N(R⁵)₂, und, wenn zwei Substituenten sich an benachbarten Kohlenstoffen befinden, sie sich verbinden können, um einen 5- oder 6gliedrigen Ring mit einem, zwei oder drei Heteroatomen, ausgewählt aus O, N und S, zu bilden, der unsubstituiert oder substituiert ist mit einem, zwei oder drei Substituenten, ausgewählt aus der Gruppe, bestehend aus: H, OH, CO₂R⁶, Br, Cl, F, I, CF₃, N(R⁷)₂, C₁-C₈-Alkoxy, C₁-C₈-Alkyl, C₂-C₈-Alkenyl, C₂-C₈-Alkinyl oder C₃-C₈-Cycloalkyl, CO(CH₂)ₙCH₃ und CO(CH₂)ₙCH₂N(R⁵)₂,
Heteroaryl definiert ist als ein 5- oder 6gliedriger aromatischer Ring mit 1, 2 oder 3 Heteroatomen, ausgewählt aus O, N und S, der unsubstituiert oder substituiert ist mit einem, zwei oder drei Substituenten, ausgewählt aus der Gruppe, bestehend aus: OH, CO₂R⁴, Br, Cl, F, I, CF₃, N(R⁵)₂, C₁-C₈-Alkoxy, C₁-C₈-Alkyl, C₂-C₈-Alkenyl, C₂-C₈-Alkinyl oder C₃-C₈-Cycloalkyl, CO(CH₂)ₙCH₃ und CO(CH₂)ₙCH₂N(R⁵)₂,
R² OR⁴ oder N(R⁵)₂ ist,
R³ ist 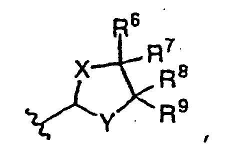
R^{3a} ist:
a) CHO,
b) -CO-C₁-C₈-Alkyl,
c) -CO-Aryl oder
d) -CO-Heteroaryl,
X und Y unabhängig sind: O, S oder NR⁵,
R⁴ verzweigtes C₃-C₈-Alkyl ist,
R⁵ ist: C₁-C₈-Alkyl oder Aryl und
R⁶, R⁷, R⁸ und R⁹ unabhängig sind: H, C₁-C₈-Alkyl und Aryl, so daß entweder R⁶ und R⁷ nicht gleich sind und/oder R⁸ und R⁹ nicht gleich sind, oder R⁶ und R⁸ oder R⁷ und R⁹ sich verbinden können, um einen 5- oder 6gliedrigen Ring zu bilden, der unsubstituiert oder substituiert ist mit einem, zwei oder drei Substituenten, ausgewählt aus der Gruppe, bestehend aus: OH, CO₂R⁴, Br, Cl, F, I, CF₃, N(R⁵)₂, C₁-C₈-Alkoxy, C₁-C₈-Alkyl, C₂-C₈-Alkenyl, C₂-C₈-Alkinyl oder C₃-C₈-Cycloalkyl, CO(CH₂)ₙCH₃, CO(CH₂)ₙCH₂N(R⁵)₂.

2. Ein Verfahren zur Herstellung einer Verbindung der Formel I: 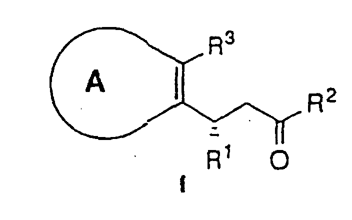 wobei die Verbindung der Formel I wie in Anspruch 1 definiert ist, umfassend die Umsetzung eines α,β-ungesättigten Esters oder Amids 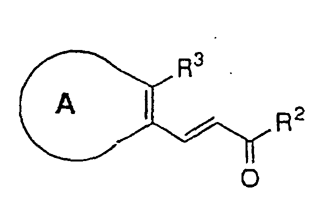 mit einer lithiumorganischen Verbindung R¹Li in Gegenwart eines aprotischen Lösungsmittels in einem Temperaturbereich von etwa -78°C bis etwa 0°C.

3. Das wie in Anspruch 2 wiedergegebene Verfahren, bei dem die Zahl der Äquivalente der lithiumorganischen Verbindung R¹Li 1 bis etwa 4 beträgt.

4. Das wie in Anspruch 3 wiedergegebene Verfahren, bei dem das aprotische Lösungsmittel ausgewählt ist aus der Gruppe, bestehend aus Tetrahydrofuran, Diethylether, Methyl-t-butylether, Benzol, Toluol, Hexan, Pentan, Dioxan und einer Mischung aus den genannten Lösungsmitteln.

5. Das wie in Anspruch 4 wiedergegebene Verfahren, bei dem der Temperaturbereich etwa -78°C bis etwa -20°C beträgt.

6. Ein Verfahren zur Herstellung einer Verbindung der Formel I: 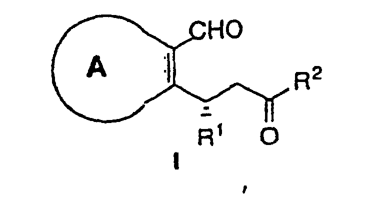 wobei die Verbindung der Formel I wie in Anspruch 1 definiert ist, umfassend die Schritte:
1) Umsetzung eines α,β-ungesättigten Esters oder Amids 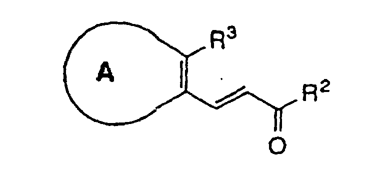 mit einer lithiumorganischen Verbindung R¹Li in Gegenwart eines aprotischen Lösungsmittels in einem Temperaturbereich von etwa -78°C bis etwa 0°C, um das Konjugataddukt zu ergeben, und
2) Entfernen des chiralen Hilfsstoffs R³ mit wäßriger Säure und Tetrahydrofuran, um die Verbindung der Formel I zu ergeben.

7. Das wie in Anspruch 6 wiedergegebene Verfahren, bei dem die Zahl der Äquivalente der lithiumorganischen Verbindung R¹Li 1 bis etwa 4 beträgt.

8. Das wie in Anspruch 7 wiedergegebene Verfahren, bei dem das aprotische Lösungsmittel ausgewählt ist aus der Gruppe, bestehend aus Tetrahydrofuran, Diethylether, Methyl-t-butylether, Benzol, Toluol, Hexan, Pentan, Dioxan und einer Mischung aus den genannten Lösungsmitteln.

9. Das wie in Anspruch 8 wiedergegebene Verfahren, bei dem der Temperaturbereich etwa -78°C bis etwa -20°C beträgt.

10. Das wie in Anspruch 9 wiedergegebene Verfahren, bei dem die wäßrige Säure wäßrige Essigsäure ist.

11. Ein Verfahren zur Herstellung von 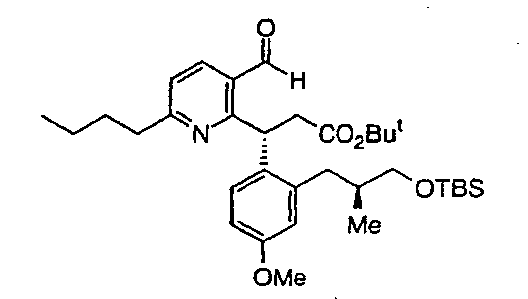 umfassend die Umsetzung eines α,β-ungesättigten Esters 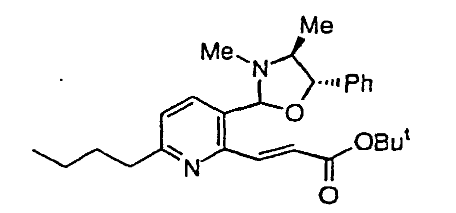 mit einer lithiumorganischen Verbindung 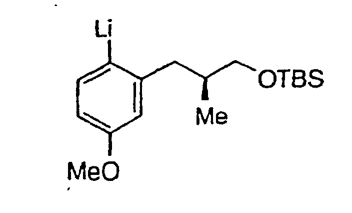 in Gegenwart eines aprotischen Lösungsmittels in einem Temperaturbereich von etwa -78°C bis etwa -20°C.

12. Das wie in Anspruch 11 wiedergegebene Verfahren, bei dem die Zahl der Äquivalente der lithiumorganischen Verbindung R¹Li 1 bis etwa 4 beträgt.

13. Das wie in Anspruch 12 wiedergegebene Verfahren, bei dem das aprotische Lösungsmittel ausgewählt ist aus der Gruppe, bestehend aus Tetrahydrofuran, Diethylether, Methyl-t-butylether, Benzol, Toluol, Pentan, Hexan, Dioxan und einer Mischung aus den genannten Lösungsmitteln.

14. Das wie in Anspruch 13 wiedergegebene Verfahren, bei dem der Temperaturbereich etwa -78°C bis etwa -50°C beträgt.

15. Das wie in Anspruch 14 wiedergegebene Verfahren, bei dem die Zahl der Äquivalente der lithiumorganischen Verbindung R¹Li 1,5 bis etwa 2,5 beträgt.

16. Das wie in Anspruch 15 wiedergegebene Verfahren, bei dem das aprotische Lösungsmittel Tetrahydrofuran ist.

17. Das wie in Anspruch 16 wiedergegebene Verfahren, bei dem der Temperaturbereich etwa -78°C bis etwa -70°C beträgt.

18. Ein Verfahren zur Herstellung eines Ketons 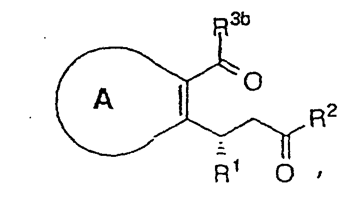 wobei 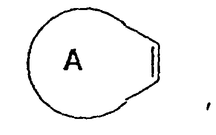 R¹ und R² wie in Anspruch 1 definiert sind und R^{3b} ist:
a) C₁-C₈-Alkyl,
b) Aryl oder
c) Heteroaryl,
wobei Aryl wie in Anspruch 1 definiert ist,
umfassend die Schritte:
1) Umsetzung eines α,β-ungesättigten Esters oder Amids 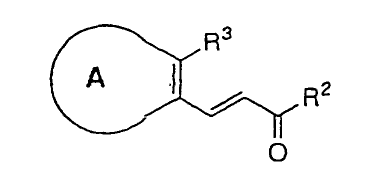 mit einer lithiumorganischen Verbindung R¹Li in Gegenwart eines aprotischen Lösungsmittels in einem Temperaturbereich von etwa -78°C bis etwa 0°C, um ein Konjugataddukt 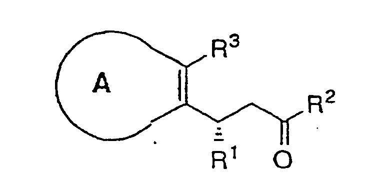 zu ergeben,
2) Entfernen des chiralen Hilfsstoffs mit wäßriger Säure und Tetrahydrofuran, um den Aldehyd 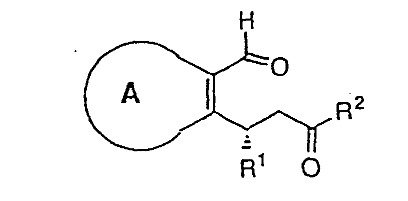 zu ergeben,
3) Umsetzung des Aldehyds mit einem Grignardreagenz oder einem lithiumorganischen Reagenz, das mit R^{3b}Z gebildet wird, wobei Z Br, Cl oder I ist, um einen Alkohol 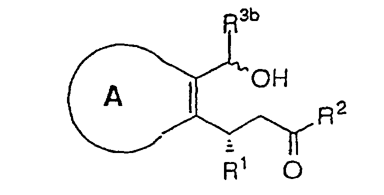 zu bilden,
4) Oxidieren des gebildeten Alkohols mit einem Oxidationsmittel, um das Keton 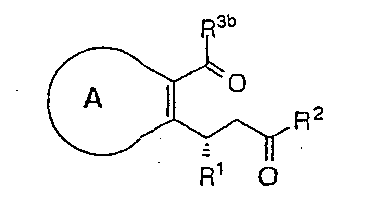 zu ergeben.

19. Das wie in Anspruch 18 wiedergegebene Verfahren, bei dem die Zahl der Äquivalente der lithiumorganischen Verbindung im ersten Schritt 1 bis etwa 4 beträgt.

20. Das wie in Anspruch 19 wiedergegebene Verfahren, bei dem das aprotische Lösungsmittel im ersten Schritt ausgewählt ist aus der Gruppe, bestehend aus Tetrahydrofuran, Diethylether, Methyl-t-butylether, Benzol, Toluol, Pentan, Hexan, Dioxan und einer Mischung aus den genannten Lösungsmitteln.

21. Das wie in Anspruch 20 wiedergegebene Verfahren, bei dem der Temperaturbereich im ersten Schritt etwa -78°C bis etwa -50°C beträgt.

22. Das wie in Anspruch 21 wiedergegebene Verfahren, bei dem die wäßrige Säure im zweiten Schritt wäßrige Essigsäure ist.

23. Das wie in Anspruch 22 wiedergegebene Verfahren, bei dem das Oxidationsmittel im vierten Schritt 4-Methylmorpholin-N-oxid und Tetrapropylammoniumperruthenat (VII) ist.

24. Ein Verfahren zur Herstellung eines Ketons der Formel: 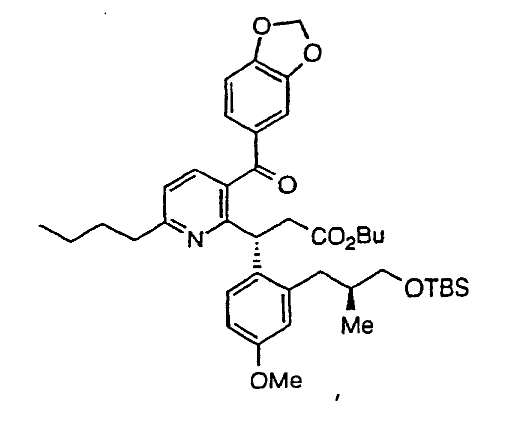 umfassend die Schritte:
1) Umsetzung eines α,β-ungesättigten Esters 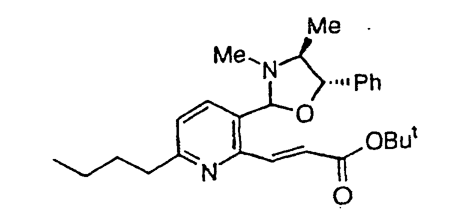 mit einer lithiumorganischen Verbindung 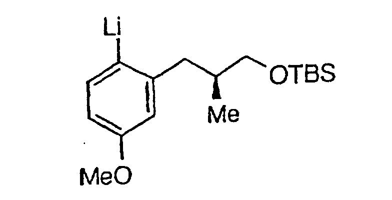 in Gegenwart eines aprotischen Lösungsmittels in einem Temperaturbereich von etwa -78°C bis etwa -20°C, um ein Konjugataddukt 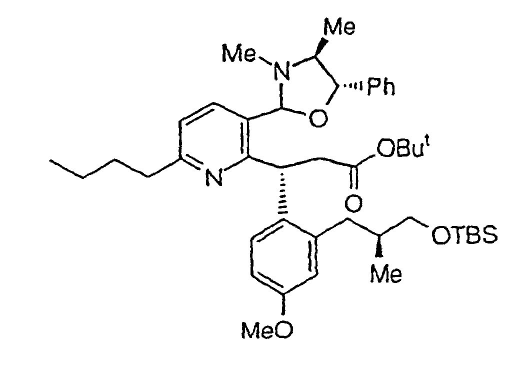 zu ergeben,
2) Entfernen des chiralen Hilfsstoffs mit wäßriger Säure und Tetrahydrofuran, um den Aldehyd 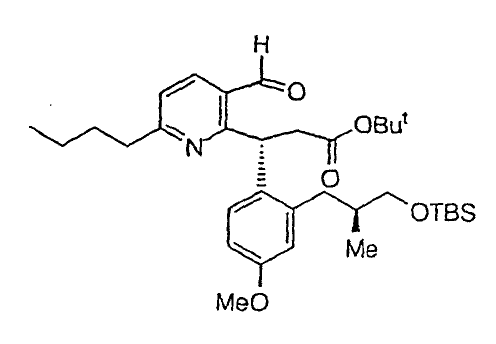 zu ergeben,
3) Umsetzung des Aldehyds mit einem Grignardreagenz oder einem lithiumorganischen Reagenz, das mit 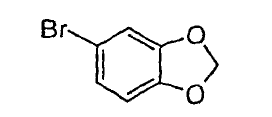 gebildet wird, um den Alkohol 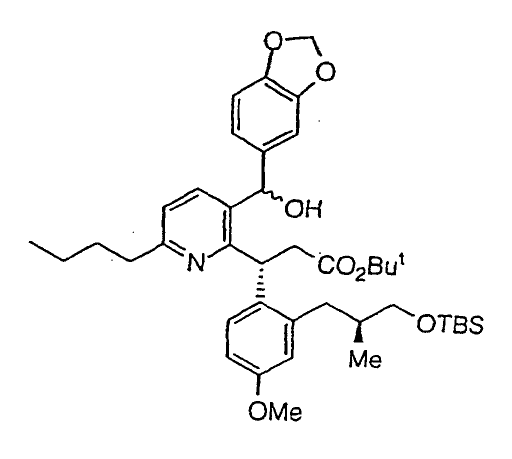 zu bilden,
4) Oxidation des gebildeten Alkohols mit einem Oxidationsmittel, um ein Keton 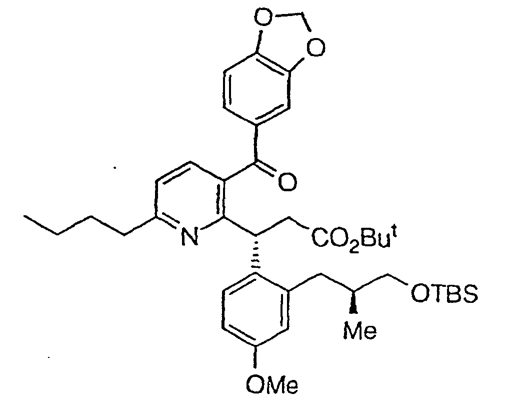 zu bilden, und
5) Umestern des Esters mit n-Butanol und einer Lewissäure, um das erwünschte Keton zu ergeben.

25. Das wie in Anspruch 24 wiedergegebene Verfahren, bei dem die Zahl der Äquivalente der lithiumorganischen Verbindung im ersten Schritt 1 bis etwa 4 beträgt.

26. Das wie in Anspruch 25 wiedergegebene Verfahren, bei dem das aprotische Lösungsmittel im ersten Schritt ausgewählt ist aus der Gruppe, bestehend aus Tetrahydrofuran, Diethylether, Methyl-t-butylether, Benzol, Toluol, Pentan, Hexan, Dioxan und einer Mischung aus den genannten Lösungsmitteln.

27. Das wie in Anspruch 26 wiedergegebene Verfahren, bei dem der Temperaturbereich im ersten Schritt etwa -78°C bis etwa -50°C beträgt.

28. Das wie in Anspruch 27 wiedergegebene Verfahren, bei dem die wäßrige Säure im zweiten Schritt wäßrige Essigsäure ist.

29. Das wie in Anspruch 28 wiedergegebene Verfahren, bei dem das Grignardreagenz im dritten Schritt 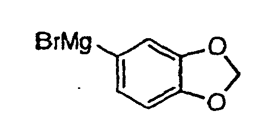 ist.

30. Das wie in Anspruch 28 wiedergegebene Verfahren, bei dem das lithiumorganische Reagenz im dritten Schritt 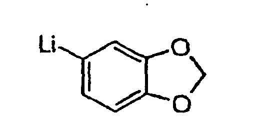 ist.

31. Das wie in Anspruch 29 wiedergegebene Verfahren, bei dem das Oxidationsmittel im vierten Schritt 4-Methylmorpholin-N-oxid und Tetrapropylammoniumperruthenat (VII) ist.

32. Das wie in Anspruch 31 wiedergegebene Verfahren, bei dem der fünfte Schritt in Gegenwart einer Lewissäure durchgeführt wird, ausgewählt aus der Gruppe, bestehend aus: Ti(OEt)₄, Ti(OiPr)₄ und Ti(OBu)₄.

## Revendications

1. Composé de formule I: 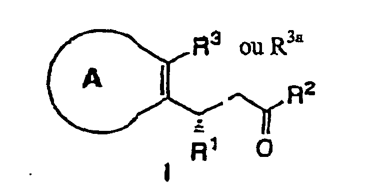 dans laquelle 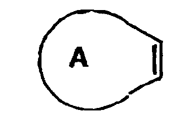 représente:
a) un groupe hétérocyclyle à 5 ou 6 chaînons contenant une, deux ou trois doubles liaisons, mais au moins une double liaison, et 1, 2 ou 3 hétéroatomes choisis parmi O, N et S, le groupe hétérocyclyle est non substitué ou substitué par un, deux ou trois substituants choisis dans le groupe constitué par OH, CO₂R⁴, Br, Cl, F, I, CF₃, N(R⁵)₂, alcoxy en C₁-C₈, alkyle en C₁-C₈, alcényle en C₂-C₈, alcynyle en C₂-C₈ ou cycloalkyle en C₃-C₈, CO(CH₂)ₙCH₃ et CO(CH₂)ₙCH₂N(R⁵)₂,
b) un groupe carbocyclyle à 5 ou 6 chaînons contenant une ou deux doubles liaisons, mais au moins une double liaison, le groupe carbocyclyle est non substitué ou substitué par un, deux ou trois substituants choisis dans le groupe constitué par OH, CO₂R⁴, Br, Cl, F, I, CF₃, N(R⁵)₂, alcoxy en C₁-C₈, alkyle en C₁-C₈, alcényle en C₂-C₈, alcynyle en C₂-C₈, ou cycloalkyle en C₃-C₈, CO(CH₂)ₙCH₃; et CO(CH₂)ₙCH₂N(R⁵)₂,
c) aryle, où aryle est tel que défini ci-dessous,
alcoxy en C₁-C₈, alkyle en C₁-C₈, alcényle en C₂-C₈, alcynyle en C₂-C₈ ou cycloalkyle en C₃-C₈ sont non substitués ou substitués par un, deux ou trois substituants choisis dans le groupe constitué par OH, CO₂R⁴, Br, Cl, F, I, CF₃, N(R⁵)₂, alcoxy en C₁-C₈, cycloalkyle en C₃-C₈, CO(CH₂)ₙCH₃ et CO(CH₂)ₙCH₂N(R⁵)₂,
n vaut 0 à 5;
R¹ est un groupe :
a) alkyle en C₁-C₈, alcényle en C₂-C₈, alcynyle en C₂-C₈, cycloalkyle en C₃-C₈,
b) aryle, ou
c) hétéroaryle;
aryle est défini comme étant phényle ou naphtyle, qui est non substitué ou substitué par un, deux ou trois substituants choisis dans le groupe constitué par OH, CO₂R⁴, Br, Cl, F, I, CF₃, N(R⁵)₂, alcoxy en C₁-C₈, alkyle en C₁-C₈, alcényle en C₂-C₈, alcynyle en C₂-C₈, ou cycloalkyle en C₃-C₈, CO(CH₂)ₙCH₃, CO(CH₂)ₙCH₂N(R⁵)₂, et, lorsque deux substituants sont situés sur des atomes de carbone adjacents, ils peuvent être reliés pour former un noyau à 5 ou 6 chaînons comportant un, deux ou trois hétéroatomes choisis parmi O, N et S, qui est non substitué ou substitué par un, deux ou trois substituants choisis dans le groupe constitué par H, OH, CO₂R⁶, Br, Cl, F, I, CF₃, N(R⁷)₂, alcoxy en C₁-C₈, alkyle en C₁-C₈, alcényle en C₂-C₈, alcynyle en C₂-C₈, ou cycloalkyle en C₃-C₈, CO(CH₂)ₙCH₃ et CO(CH₂)ₙCH₂N(R⁵)₂,
hétéroaryle est défini comme étant un noyau aromatique à 5 ou 6 chaînons contenant 1, 2 ou 3 hétéroatomes choisis parmi O, N et S, qui est non substitué ou substitué par un, deux ou trois substituants choisis dans le groupe constitué par OH, CO₂R⁴, Br, Cl, F, I, CF₃, N(R⁵)₂, alcoxy en C₁-C₈, alkyle en C₁-C₈, alcényle en C₂-C₈, alcynyle en C₂-C₈, ou cycloalkyle en C₃-C₈, CO(CH₂)ₙCH₃ et CO(CH₂)ₙCH₂N(R⁵)₂.
R² est OR⁴ ou N(R⁵)₂;
R³ est: 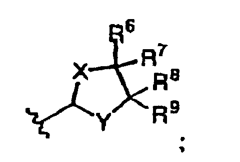
R^{3a} est un groupe:
a) CHO,
b) -CO-alkyle en C₁-C₈,
c) -CO-aryle, ou
d) -CO-hétéroaryle;
X et Y sont indépendamment l'un de l'autre O, S ou NR⁵;
R⁴ est un groupe alkyle ramifié en C₁-C₈;
R⁵ est un groupe alkyle en C₁-C₈ ou aryle; et
R⁶, R⁷, R⁸ et R⁹ sont indépendamment l'un de l'autre II ou un groupe alkyle en C₁-C₈ et aryle, de telle sorte que soit les radicaux R⁶ et R⁷ ne sont pas identiques et/ou R⁸ et R⁹ ne sont pas identiques, soit les radicaux R⁶ et R⁸ ou R⁷ et R⁹ peuvent être reliés pour former un noyau à 5 ou 6 chaînons, qui est non substitué ou substitué par un, deux ou trois substituants choisis dans le groupe constitué par OH, CO₂R⁴, Br, CI, F, I, CF₃, N(R⁵)₂, alcoxy en C₁-C₈, alkyle en C₁-C₈, alcényle en C₂-C₈, alcynyle en C₂-C₈, ou cycloalkyle en C₃-C₈, CO(CH₂)ᵤCH₃, CO(CH₂)ₙCH₂N(R⁵)₂.

2. Procédé de préparation d'un composé de formule I: 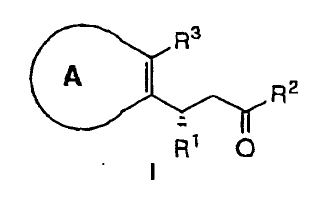 dans laquelle le composé de formule I est tel que défini dans la revendication 1, comprenant le fait de faire réagir un ester ou amide α,β-insaturé 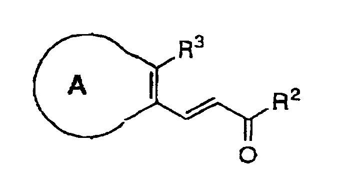 avec un composé organolithium, R¹Li, en présence d'un solvant aprotique dans une gamme de températures d'environ -78°C à environ 0°C.

3. Procédé selon la revendication 2, dans lequel le nombre d'équivalents du composé organolithium, R¹Li, est de 1 à environ 4.

4. Procédé selon la revendication 3, dans lequel le solvant aprotique est choisi dans le groupe constitué par le tétrahydrofurane, l'éther diéthylique, le méthyl-t-butyléther, le benzéne, le toluène, l'hexane, le pentane, le dioxane et un mélange desdits solvants.

5. Procédé selon la revendication 4, dans lequel la gamme de températures est d'environ -78°C à environ -20°C.

6. Procédé de préparation d'un composé de formule I: 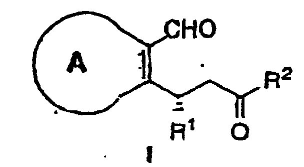 dans laquelle le composé de formule I est tel que défini dans la revendication 1, comprenant les étapes consistant à :
1) faire réagir un ester ou amide α,β-insaturé 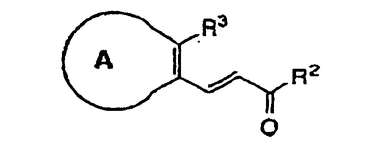 avec un composé organolithium, R¹Li, en présence d'un solvant aprotique, dans une gamme de températures d'environ -78°C à environ 0°C, pour obtenir le composé d'addition conjugué ; et
2) éliminer l'auxiliaire chiral, R³, avec un acide aqueux et du tétrahydrofurane, pour obtenir le composé de formule I.

7. Procédé selon la revendication 6, dans lequel le nombre d'équivalents du composé organolithium, R¹Li, est de 1 à environ 4.

8. Procédé selon la revendication 7, dans lequel le solvant aprotique est choisi dans le groupe constitué par le tétrahydrofurane, l'éther diéthylique, le méthyl-t-butyléther, le benzène, le toluène, l'hexane, le pentane, le dioxane et un mélange desdits solvants.

9. Procédé selon la revendication 8, dans lequel la gamme de températures est d'environ -78°C à environ -20°C.

10. Procédé selon la revendication 9, dans lequel l'acide aqueux est de l'acide acétique aqueux.

11. Procédé de préparation de 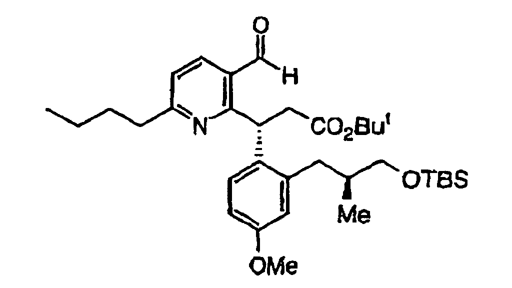 comprenant le fait de faire réagir un ester α,β-insaturé 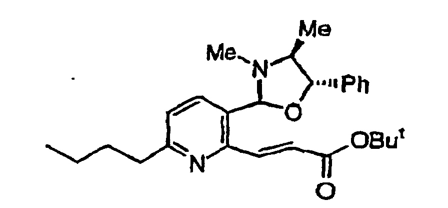 avec un composé organolithium 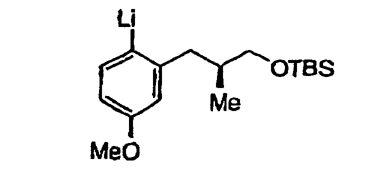 en présence d'un solvant aprotique, dans une gamme de températures d'environ -78°C à environ -20°C.

12. Procédé selon la revendication 11, dans lequel le nombre d'équivalents du composé organolithium, R¹Li, est de 1 à environ 4.

13. Procédé selon la revendication 12, dans lequel le solvant aprotique est choisi dans le groupe constitué par le tétrahydrofurane, l'éther diéthylique, le méthyl-t-butyléther, le benzène, le toluène, le pentane, l'hexane, le dioxane et un mélange desdits solvants.

14. Procédé selon la revendication 13, dans lequel la gamme de températures est d'environ -78°C à environ -50°C.

15. Procédé selon la revendication 14, dans lequel le nombre d'équivalents du composé organolithium, R¹Li, est de 1,5 à environ 2,5.

16. Procédé selon la revendication 15, dans lequel le solvant aprotique est le tétrahydrofurane.

17. Procédé selon la revendication 16, dans lequel la gamme de températures est d'environ -78°C à environ -70°C.

18. Procédé de préparation d'une cétone 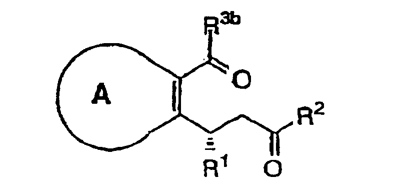 dans laquelle 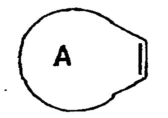 R¹ et R² sont tels que définis dans la revendication 1; et
R^{3b} est un groupe :
a) alkyle en C₁-C₈,
b) aryle, ou
c) hétéroaryle;
où aryle est tel que défini dans la revendication 1;
comprenant les étapes consistant à:
1) faire réagir un ester ou amide α,β-insaturé 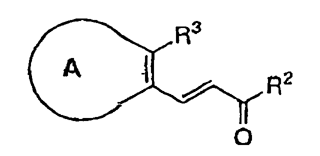 avec un composé organolithium, R¹Li, en présence d'un solvant aprotique, dans une gamme de températures d'environ -78°C à environ 0°C, pour obtenir un composé d'addition conjugué 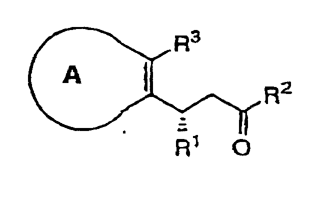
2) éliminer l'auxiliaire chiral avec un acide aqueux et du tétrahydrofurane pour obtenir l'aldéhyde 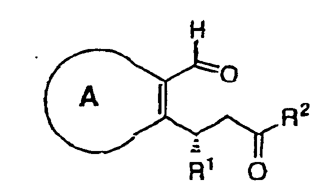
3) faire réagir l'aldéhyde avec un réactif de Grignard ou un réactif organolithium formé avec R^{3b}Z, où Z est Br, Cl ou I, pour former un alcool 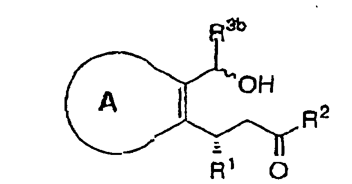
4) oxyder l'alcool formé avec un agent oxydant pour obtenir la cétone

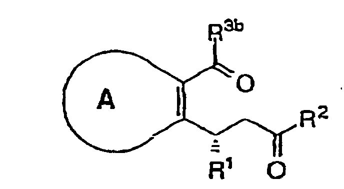

19. Procédé selon la revendication 18, dans lequel le nombre d'équivalents du composé organolithium dans la première étape est de 1 à environ 4.

20. Procédé selon la revendication 19, dans lequel le solvant aprotique dans la première étape est choisi dans le groupe constitué par le tétrahydrofurane, l'éther diéthylique, le méthyl-t-butyléther, le benzène, le toluène, le pentane, l'hexane, le dioxane et un mélange desdits solvants.

21. Procédé selon la revendication 20, dans lequel la gamme de températures dans la première étape est d'environ -78°C à environ -50°C.

22. Procédé selon la revendication 21, dans lequel l'acide aqueux dans la deuxième étape est de l'acide acétique aqueux.

23. Procédé selon la revendication 22, dans lequel l'agent oxydant dans la quatrième étape est le N-oxyde de 4-méthylmorpholine et le perruthénate (VII) de tétrapropylammonium.

24. Procédé de préparation d'une cétone de formule: 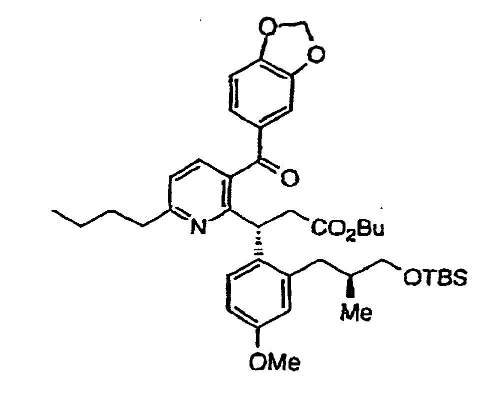 comprenant les étapes consistant à:
1) faire réagir un ester α,β-insaturé 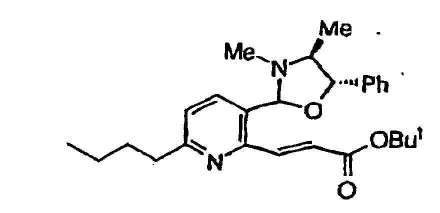 avec un composé organolithium 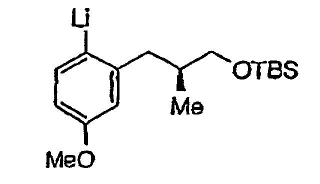 en présence d'un solvant aprotique dans une gamme de températures d'environ -78°C à environ -20°C pour obtenir un composé d'addition conjugué 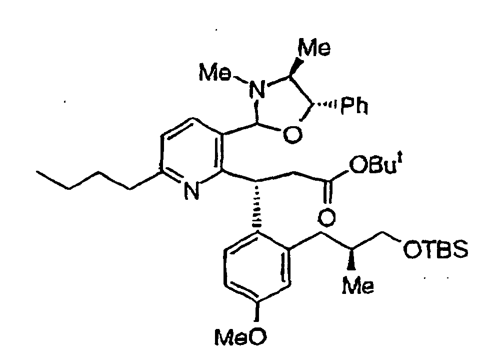
2) éliminer l'auxiliaire chiral avec un acide aqueux et du tétrahydrofurane pour obtenir l'aldéhyde 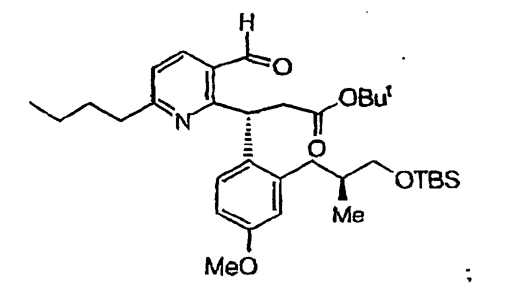
3) faire réagir l'aldéhyde avec un réactif de Grignard ou un réactif organolithium formé avec 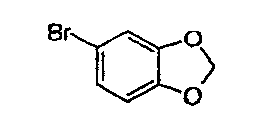 pour former l'alcool 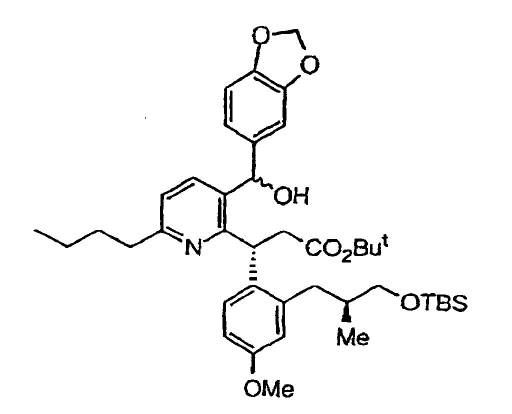
4) oxyder l'alcool formé avec un agent oxydant pour obtenir une cétone 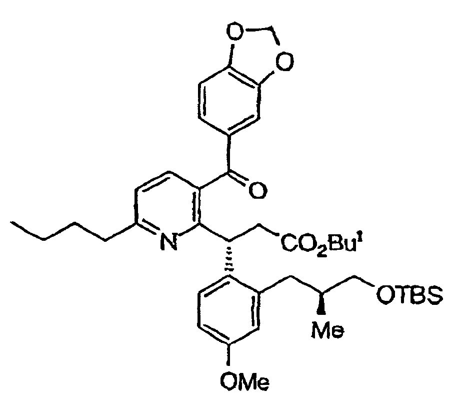 et
5) transestérifier l'ester avec du n-butanol et un acide Lewis pour obtenir la cétone souhaitée.

25. Procédé selon la revendication 24, dans lequel le nombre d'équivalents du composé organolithium dans la première étape est de 1 à environ 4.

26. Procédé selon la revendication 25, dans lequel le solvant aprotique dans la première étape est choisi dans le groupe constitué par le tétrahydrofurane, l'éther diéthylique, le méthyl-t-butyléther, le benzène, le toluène, le pentane, l'hexane, le dioxane et un mélange desdits solvants.

27. Procédé selon la revendication 26, dans lequel la gamme de températures dans la première étape est d'environ -78°C à environ -50°C.

28. Procédé selon la revendication 27, dans lequel l'acide aqueux dans la deuxième étape est de l'acide acétique aqueux.

29. Procédé selon la revendication 28, dans lequel le réactif de Grignard dans la troisième étape est

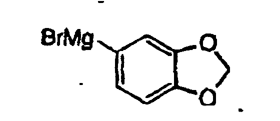

30. Procédé selon la revendication 28, dans lequel le réactif organolithium dans la troisième étape est

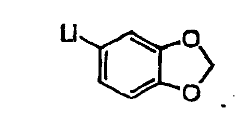

31. Procédé selon la revendication 29, dans lequel l'agent oxydant dans la quatrième étape est le N-oxyde de 4-méthylmorpholine et le perruthénate (VII) de tétrapropylammonium.

32. Procédé selon la revendication 31, dans lequel la cinquième étape est réalisée en présence d'un acide de Lewis choisi dans le groupe constitué par Ti(OEt)₄, Ti(OiPr)₄ et Ti(OBu)₄.
